# EUROPEAN PATENT SPECIFICATION

(11) **EP 4 202 527 B1**
(45) Date of publication and mention of the grant of the patent: **14.10.2026**
(21) Application number: 21861046.7
(22) Date of filing: 19.07.2021
(51) Int. Cl.: G02B 23/24, A61B 1/045, A61B 1/00, A61B 1/05, G06V 10/25, G02B 23/00

(54) **IMAGE PROCESSING DEVICE**
BILDVERARBEITUNGSVORRICHTUNG
DISPOSITIF DE TRAITEMENT D'IMAGE

(30) Priority: 24.08.2020 JP 2020140944
(43) Date of publication of application: 28.06.2023
(73) Proprietor: FUJIFILM Corporation, Tokyo 106-8620 (JP)
(72) Inventor: USUDA Toshihiro, Tokyo 106-8620 (JP)
(74) Representative: Dehns Germany Partnerschaft mbB
(86) International application number: PCT/JP2021/026905
(87) International publication number: WO 2022/044617

(56) References cited:
- WO-A1-2012/165204
- WO-A1-2014/103237
- WO-A1-2018/235179
- WO-A1-2018/235179
- WO-A1-2019/116592
- WO-A1-2020/036224
- JP-A- 2009 226 072
- US-A1- 2012 182 409
- US-A1- 2015 282 887
- US-A1- 2016 038 004

## Description

### BACKGROUND OF THE INVENTION

### 1. Field of the Invention

The present invention relates to an image processing apparatus, and more specifically to an image processing apparatus for processing an image captured with an endoscope having a distal end from which an instrument is protrudable.

### 2. Description of the Related Art

As a technique for supporting an examination using an endoscope, a technique is known for automatically detecting a region of interest such as a lesion from an image captured with an endoscope by image processing and for providing a notification.

International Publication No. WO2017/002184A proposes that, to efficiently support an examination, an action taken by an operator of an endoscope be determined from an image captured with the endoscope and an image on which a predetermined action such as a treatment has been performed be excluded from a detection target of a region of interest. That is, if a treatment or the like has been performed, it is likely that the region of interest has already been found, and there seems to be less need for detection using image processing. Thus, such an image is excluded from the detection target to achieve efficiency.

US 2016/038004 discloses an endoscope apparatus and a method for operating the endoscope apparatus. WO 2018/235179 discloses an image processing device, an endoscope device, a method for operating image processing device, and an image processing program. US 2012/182409 discloses an endoscope system and a low visibility determining method.

### SUMMARY OF THE INVENTION

An examination using an endoscope may involve tissue sampling (biopsy). Tissue sampling is performed through a forceps port included in a tip part of the endoscope. During tissue sampling, it is expected to notify an operator of a lesion, which is a target, at an appropriate timing to support the operator.

In International Publication No. WO2017/002184A, however, a lesion, which is a target, is not detected during the treatment, resulting in a disadvantage in that it is difficult to appropriately support an operator.

The present invention has been made in view of such circumstances, and an object thereof is to provide an image processing apparatus that can appropriately support a treatment using an instrument.

The invention is defined by features of independent claim 1. Preferred embodiments are defined in the dependent claims 2-14.

According to the present invention, it is possible to appropriately support a treatment using an instrument.

### BRIEF DESCRIPTION OF THE DRAWINGS

Fig. 1 is a block diagram illustrating an example of a system configuration of an endoscope system to which the present disclosure is applied;
Fig. 2 is a diagram illustrating an example of an endoscope;
Fig. 3 is a perspective view illustrating an example configuration of a distal end of an insertion section of the endoscope;
Fig. 4 is a block diagram of a function implemented by an image processing apparatus;
Fig. 5 is a diagram illustrating an example of a display image in a case where a treatment tool is protruded from a forceps port;
Fig. 6 is a diagram illustrating an example of a display image;
Fig. 7 is a diagram illustrating an example of a display image;
Fig. 8 is a flowchart illustrating a processing procedure for displaying an endoscopic image on a display by the image processing apparatus;
Figs. 9A and 9B are diagrams illustrating a first modification of display images;
Figs. 10A and 10B are diagrams illustrating a second modification of display images;
Fig. 11 is a block diagram illustrating an example of a system configuration of an ultrasonic endoscope system to which the present disclosure is applied;
Fig. 12 is a diagram illustrating an example of an ultrasonic endoscope;
Fig. 13 is a perspective view illustrating an example configuration of a distal end of an insertion section of the ultrasonic endoscope;
Fig. 14 is a block diagram of a function implemented by an image processing apparatus according to a second embodiment;
Fig. 15 is a diagram illustrating an example of a display image in a case where a treatment tool is protruded from a treatment tool protruding port;
Fig. 16 is a diagram illustrating an example of a display image;
Fig. 17 is a diagram illustrating an example of a display image;
Fig. 18 is a flowchart illustrating a processing procedure for displaying an endoscopic image on a display by the image processing apparatus;
Fig. 19 is a diagram illustrating a first modification of the display image;
Fig. 20 is a diagram illustrating a second modification of the display image;
Fig. 21 is a block diagram of a function implemented by an image processing apparatus according to a third embodiment;
Fig. 22 is a diagram illustrating an example of a display image;
Fig. 23 is a diagram illustrating an example of a display image;
Fig. 24 is a flowchart illustrating a processing procedure for displaying an endoscopic image on a display by the image processing apparatus;
Fig. 25 is a block diagram of a function implemented by an image processing apparatus according to a fourth embodiment; and
Fig. 26 is a flowchart illustrating a processing procedure for displaying an endoscopic image on a display by the image processing apparatus.

### DESCRIPTION OF THE PREFERRED EMBODIMENTS

Preferred embodiments of the present invention will be described in detail hereinafter with reference to the accompanying drawings.

### First Embodiment

### System Configuration

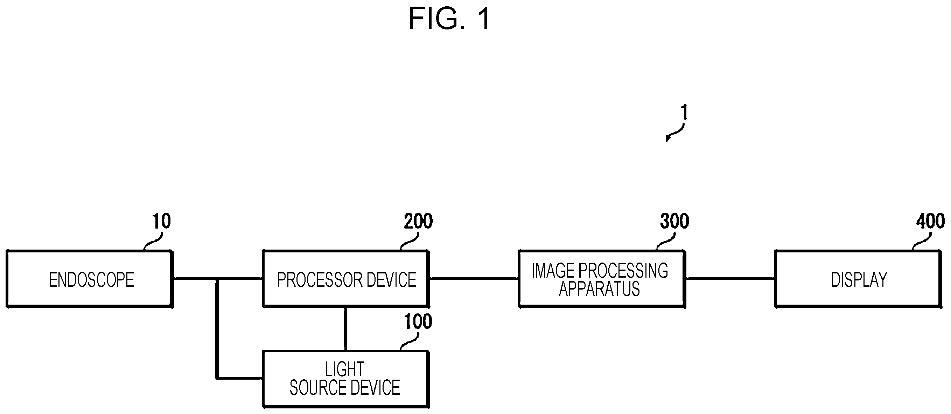

Fig. 1 is a block diagram illustrating an example of a system configuration of an endoscope system to which the present disclosure is applied.

As illustrated in Fig. 1, an endoscope system 1 according to this embodiment includes an endoscope 10, a light source device 100, a processor device 200, an image processing apparatus 300, and a display 400.

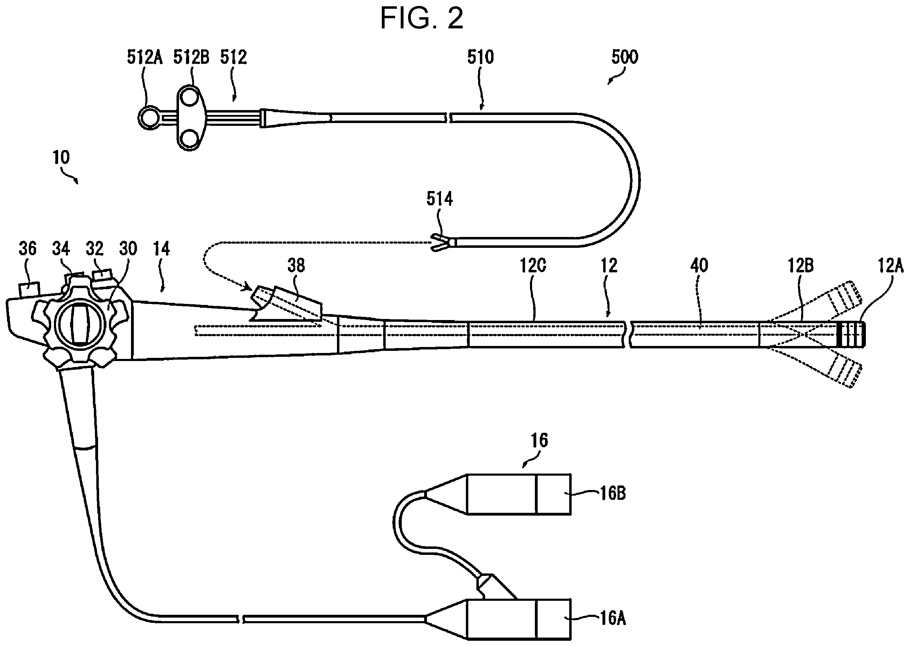

Fig. 2 is a diagram illustrating an example of the endoscope.

The endoscope 10 is a soft endoscope (electronic endoscope) and is an endoscope having a distal end from which a treatment tool is protrudable. As illustrated in Fig. 2, the endoscope 10 is mainly constituted by an insertion section 12, an operation section 14, and a connection section 16.

The insertion section 12 is a portion to be inserted into a body cavity. The insertion section 12 is constituted by, in order from the distal end side thereof, a tip part 12A, a bending part 12B that is bendable, and a soft part 12C having flexibility.

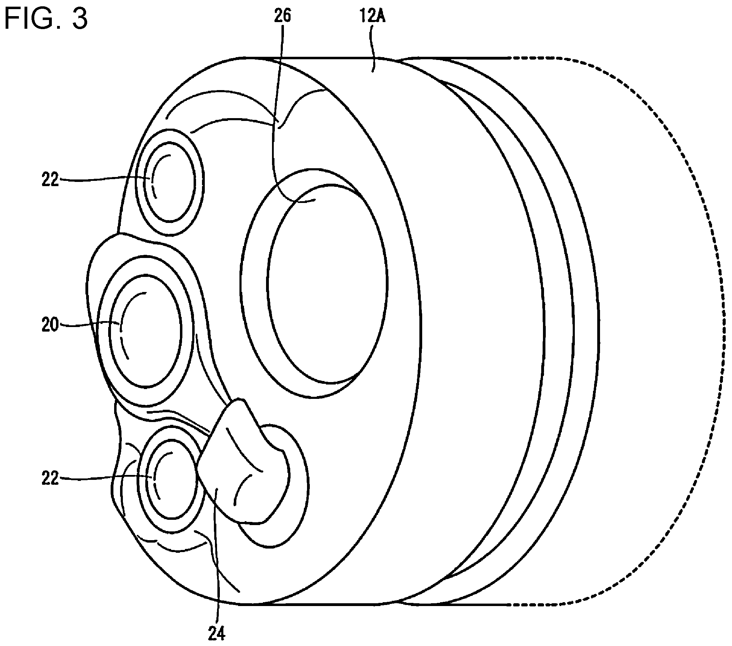

Fig. 3 is a perspective view illustrating an example configuration of the distal end of the insertion section of the endoscope.

As illustrated in Fig. 3, the tip part 12A includes, on an end surface thereof, an observation window 20, an illumination window 22, a nozzle 24, a forceps port 26, and so on.

The observation window 20 is a window for observation. An imaging unit is included on the inside of the observation window 20. The imaging unit is configured to include an imaging optical system and an image sensor. Examples of the image sensor include a color CMOS (Complementary Metal-Oxide Semiconductor) image sensor having a predetermined color filter arrangement (for example, a Bayer arrangement or the like), and a color CCD (Charge Coupled Device) image sensor.

The illumination window 22 is a window for illumination. Illumination light supplied from the light source device 100 is emitted through the illumination window 22. As illustrated in Fig. 3, the endoscope 10 according to this embodiment includes two illumination windows 22.

The nozzle 24 selectively ejects a liquid (for example, water) and a gas (for example, air) toward the observation window 20. For example, if the observation window 20 is contaminated, the contamination is washed off with the liquid or gas ejected from the nozzle 24.

The forceps port 26 is an outlet of a treatment tool 500, such as forceps. The treatment tool 500, which is inserted from a forceps insertion port 38 included in the operation section 14, protrudes from the forceps port 26. The treatment tool 500 is an example of an instrument.

The bending part 12B bends upward, downward, or to the right or left in response to an operation of an angle knob 30 included in the operation section 14. As a result, the tip part 12A can be directed in a desired direction.

As illustrated in Fig. 2, the operation section 14 is a portion to be gripped by an operator (user) to operate the endoscope 10. The operation section 14 includes various operation members. The operation section 14 includes, for example, the angle knob 30 for bending operation of the bending part 12B, an air/water supply button 32 for air/water supply operation, a suction button 34 for suction operation, a shutter release button 36 for capturing a still image, and so on.

As illustrated in Fig. 2, the operation section 14 further includes the forceps insertion port 38 from which the treatment tool 500, such as forceps, is to be inserted. The treatment tool 500, which is inserted from the forceps insertion port 38, passes through a forceps channel 40 included inside the insertion section 12 and protrudes from the forceps port 26 at the distal end.

The connection section 16 is a portion for connecting the endoscope 10 to the light source device 100 and the processor device 200. The connection section 16 is constituted by a flexible cord. The connection section 16 includes, at a distal end thereof, a connector 16A for connecting to the light source device 100 and a connector 16B for connecting to the processor device 200.

The light source device 100 includes a light source and supplies light from the light source to the endoscope 10 as illumination light. The illumination light supplied to the endoscope 10 is emitted from the illumination window 22 at the distal end through a light guide (not illustrated). The light from the light source is white light, for example.

The processor device 200 performs a process of capturing an imaging signal output from the endoscope 10, performing predetermined signal processing, and generating an observation image (endoscopic image) obtained by the endoscope 10. Further, the processor device 200 performs overall control of the entire system. The processor device 200 is constituted by, for example, a computer including a CPU (Central Processing Unit), a ROM (Read Only Memory), a RAM (Random Access Memory), and so on. In the processor device 200, the CPU executes a predetermined program to implement a function of generating an endoscopic image, a function of performing overall control of the entire system, and so on. The ROM stores various programs to be executed by the CPU, data necessary for control, processing, and the like, and so on. The RAM provides a working memory space for the CPU.

The image processing apparatus 300 performs a process of acquiring the endoscopic image output from the processor device 200 and displaying the endoscopic image on the display 400. Further, the image processing apparatus 300 performs a process of supporting the operator in a predetermined treatment using the treatment tool 500 through the display 400. Specifically, in tissue sampling, the image processing apparatus 300 displays predetermined information on the display 400 to help the operator easily sample the target (such as a lesion). This feature will be described below. The image processing apparatus 300 is constituted by, for example, a computer including a CPU, a ROM, a RAM, and so on. The image processing apparatus 300 functions as an image processing apparatus in response to a predetermined program being executed by the CPU. The ROM stores various programs to be executed by the CPU, data necessary for various types of processing and control, and so on. The RAM provides a working memory space for the CPU.

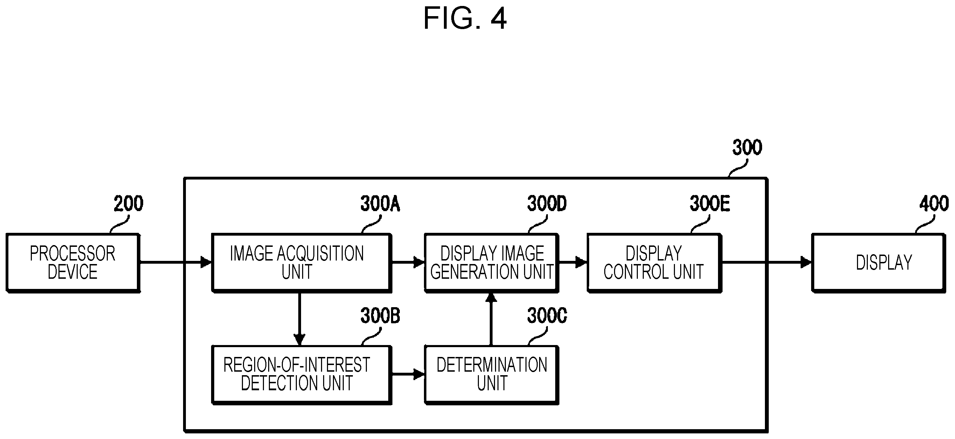

Fig. 4 is a block diagram of a function implemented by the image processing apparatus.

As illustrated in Fig. 4, the image processing apparatus 300 may have the functions of an image acquisition unit 300A, a region-of-interest detection unit 300B, a determination unit 300C, a display image generation unit 300D, and a display control unit 300E. These functions are implemented by the CPU executing a predetermined program (image processing program).

The image acquisition unit 300A acquires an endoscopic image from the processor device 200. The endoscopic image is a moving image. The image acquisition unit 300A sequentially acquires images of respective frames constituting the moving image.

The region-of-interest detection unit 300B detects a region of interest from the image of each of the frames acquired by the image acquisition unit 300A. The term "region of interest", as used herein, refers to a region on which the treatment is to be performed. In the case of tissue sampling, the region of interest is a lesion portion, which is the target. The region-of-interest detection unit 300B detects the region of interest from within the endoscopic image by, for example, image recognition. The image recognition can be performed using, for example, an image recognition model generated by machine learning (such as deep learning). Any other known method can be used.

The region of interest is detected by identification of the position of the region of interest in the image. The position is acquired as, for example, information on the position of the pixel at the center or centroid of the region of interest present in the endoscopic image.

Upon detection of a region of interest, the determination unit 300C determines from the endoscopic image whether the region of interest is located at a position where the treatment is easily performed with the treatment tool 500. A position where the treatment is easily performed with the treatment tool 500 is a position reachable by the treatment tool 500. Accordingly, the determination unit 300C determines from the image whether the region of interest is present at a position reachable by the treatment tool 500. The determination is performed in the following way.

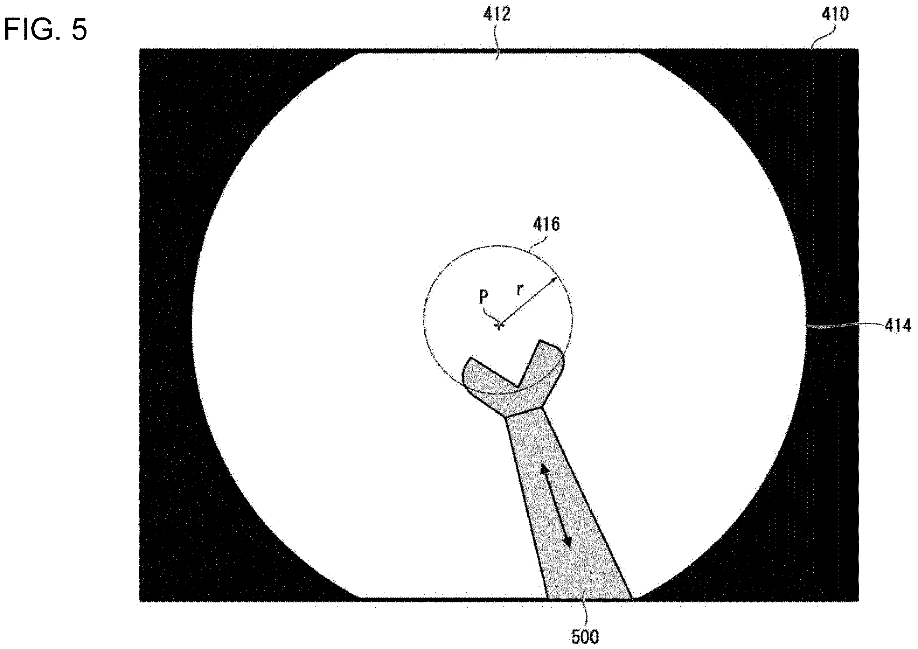

Fig. 5 is a diagram illustrating an example of a display image (an image to be displayed on the display) in a case where the treatment tool is protruded from the forceps port.

As illustrated in Fig. 5, a display image 410 is constituted by a rectangular image having a predetermined aspect ratio. An endoscopic image 412 is displayed in a predetermined display region 414 set in the display image 410. In the illustrated example, a region of a circle whose upper and lower portions are cut away is set as the display region 414 for the endoscopic image 412.

When the treatment tool 500 is protruded from the forceps port 26, the treatment tool 500 appears in the endoscopic image 412. Since the positional relationship between the forceps port 26 and the observation window 20 is fixed, the position at which the treatment tool 500 appears is always constant. Its protruding direction (the direction indicated by an arrow in Fig. 5) is also constant. Thus, a range reachable by the treatment tool 500 in the endoscopic image can be determined in advance. The range can be determined by, for example, as illustrated in Fig. 5, a circle 416 centered at a predetermined reference point P and having a radius r. When the treatment tool 500 is protruded from the forceps port 26, the distal end thereof is typically located at or near the center of the endoscopic image 412. Thus, the center of the endoscopic image 412 can be set as the reference point P to determine the range reachable by the treatment tool 500.

The determination unit 300C determines whether the region of interest is present within the range of the circle 416 centered at the center (reference point P) of the endoscopic image 412 and having the radius r to determine whether the region of interest is present at a position reachable by the treatment tool 500. In other words, the determination unit 300C determines whether the distance from the center (reference point P) of the endoscopic image 412 to the region of interest is less than or equal to r (less than or equal to a first threshold value) to determine whether the region of interest is present at a position reachable by the treatment tool 500. In this case, r is an example of the first threshold value. If the region of interest is present within the range of the circle 416 centered at the center (reference point P) of the endoscopic image 412 and having the radius r (if the distance from the center (reference point P) of the endoscopic image 412 to the region of interest is less than or equal to r), it is determined that the region of interest is present at a position reachable by the treatment tool 500. By contrast, if the region of interest is not present within the range of the circle 416 centered at the center (reference point P) of the endoscopic image 412 and having the radius r (if the distance from the center (reference point P) of the endoscopic image 412 to the region of interest exceeds r), it is determined that the region of interest is not present at a position reachable by the treatment tool 500.

The display image generation unit 300D generates a display image to be displayed on the display 400 from the endoscopic image acquired by the image acquisition unit 300A. The display image generation unit 300D generates the display image on the basis of a determination result of the determination unit 300C. That is, if the determination unit 300C determines that the region of interest is present at a position reachable by the treatment tool 500, the display image generation unit 300D changes the display image and notifies the operator that the region of interest is present at a position reachable by the treatment tool 500. Specifically, the display image generation unit 300D changes the display of the display region 414 for the endoscopic image 412.

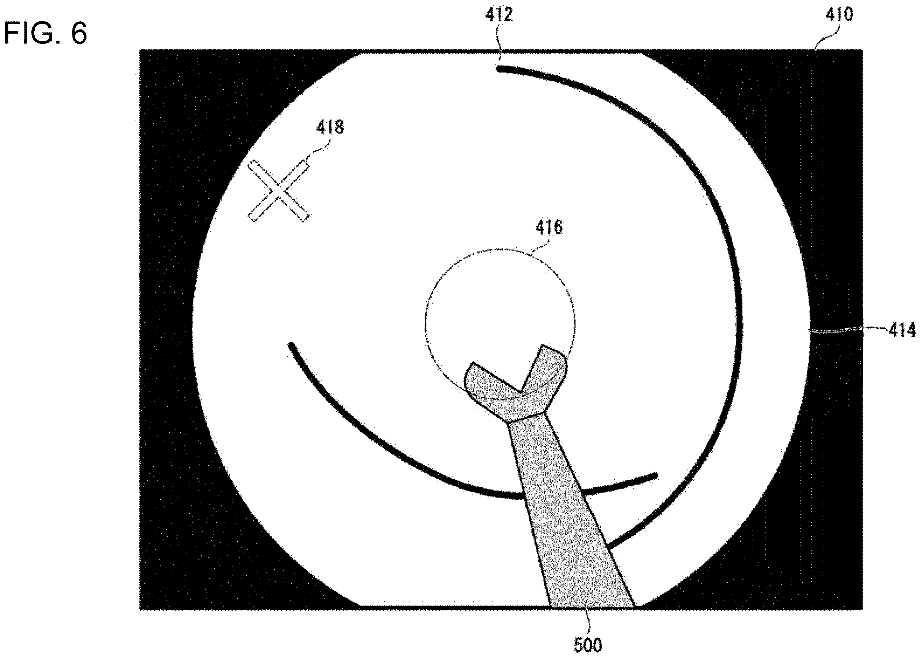

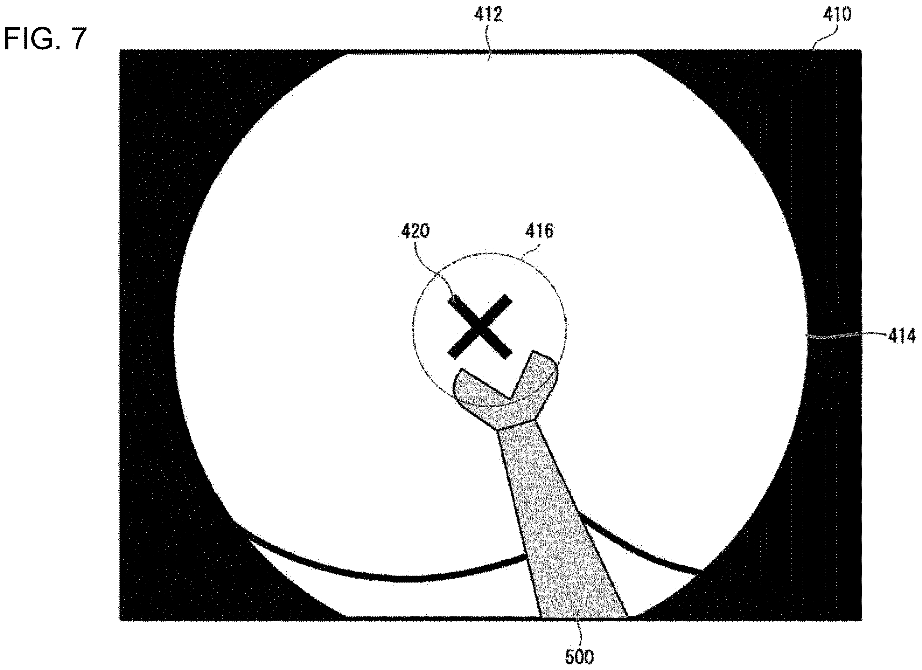

Fig. 6 and Fig. 7 are diagrams illustrating an example of the display image. Fig. 6 illustrates an example of a case where the region of interest is not present at a position reachable by the treatment tool 500. Fig. 7 illustrates an example of a case where the region of interest is present at a position reachable by the treatment tool 500.

In a case where the region of interest is not present at a position reachable by the treatment tool 500, as illustrated in Fig. 6, only the endoscopic image 412 is displayed in the display region 414 for the endoscopic image 412. In Fig. 6, a mark 418 of a cross (X) indicated by broken lines indicates the position of the region of interest. In the example illustrated in Fig. 6, since the position of the region of interest (the position of the mark 418) is outside the range of the circle 416, it is determined that the region of interest is not present at a position reachable by the treatment tool 500. In Fig. 6, the circle 416 indicating the range reachable by the treatment tool 500 and the mark 418 indicating the position of the region of interest are displayed, for convenience of description; however, these are not actually displayed in the display image 410.

By contrast, in a case where the region of interest is present at a position reachable by the treatment tool 500, as illustrated in Fig. 7, in the display region 414 for the endoscopic image 412, a mark 420 of a cross (X) indicating the position of the region of interest is displayed superimposed on the endoscopic image 412. In Fig. 7, the circle 416 indicating the range reachable by the treatment tool 500 is displayed, for convenience of description; however, the circle 416 is not actually displayed in the display image 410.

The mark 420 indicating the position of the region of interest is displayed only when the region of interest is present at a position reachable by the treatment tool 500, thereby making it possible to appropriately notify the operator of the timing of the treatment. The mark 420 is an example of a geometric figure indicating the region of interest.

The display control unit 300E causes the display 400 to display the display image generated by the display image generation unit 300D.

The display 400 is constituted by, for example, a liquid crystal display, an organic EL display (organic EL: Organic ElectroLuminescent, OEL), or the like.

### Operation

Here, tissue sampling using biopsy forceps will be described as an example.

As illustrated in Fig. 2, the biopsy forceps (treatment tool) 500 has an insertion section 510 and an operation section 512. The insertion section 510 has flexibility and has a tip claw portion 514 at a distal end thereof. The operation section 512 has a handle 512A and a slider 512B. The slider 512B is slid back and forth to open and close the tip claw portion 514. In tissue sampling, the target (such as a lesion) is grabbed with the tip claw portion 514 and sampled.

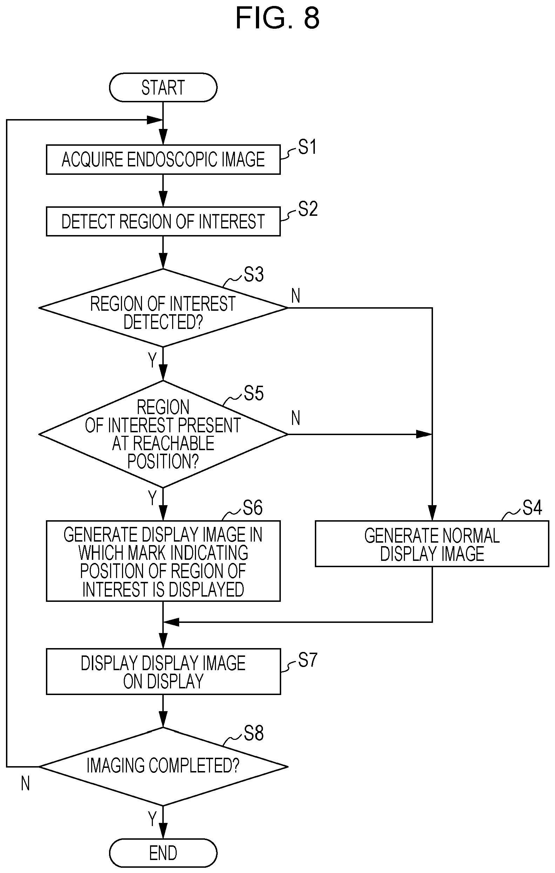

Fig. 8 is a flowchart illustrating a processing procedure (image processing method) not according to the invention for displaying an endoscopic image on the display by the image processing apparatus.

First, an endoscopic image is acquired from the processor device 200 (step S1). The endoscopic image is acquired frame by frame sequentially.

Then, a region of interest is detected from the acquired endoscopic image (step S2). The region of interest used here is a lesion portion from which the tissue is to be sampled.

Then, it is determined whether the region of interest has been detected on the basis of the detection result of the region of interest (step S3).

If it is determined that the region of interest has not been detected, a normal display image is generated (step S4). Then, the generated display image is displayed on the display 400 (step S7). The normal display image is an image in which a mark indicating the position of the region of interest is not displayed in the endoscopic image (an image with the display of the mark set to off) (see Fig. 6).

On the other hand, if it is determined that the region of interest has been detected, it is further determined whether the region of interest is present at a position reachable by the biopsy forceps 500 (step S5).

If it is determined that the region of interest is not present at a position reachable by the biopsy forceps 500, as in the case where the region of interest has not been detected, a normal display image is generated (step S4), and the generated display image is displayed on the display 400 (step S7).

On the other hand, if it is determined that the region of interest is present at a position reachable by the biopsy forceps 500, a display image is generated in which the mark indicating the position of the region of interest is displayed (step S6). Then, the generated display image is displayed on the display 400 (step S7). As illustrated in Fig. 7, the display image in which the mark indicating the position of the region of interest is displayed is an image in which the mark 420 indicating the position of the region of interest is displayed superimposed on the endoscopic image 412 (an image with the display of the mark set to on).

Thereafter, it is determined whether imaging with the endoscope 10 is completed (step S8). In response to the completion of imaging, the display process ends. The completion of imaging is determined based on, for example, whether an image of the subsequent frame is input. If imaging is not completed, the process returns to step S1, and the series of processing operations described above is performed.

In the endoscope system 1 according to this embodiment, as described above, when the region of interest is located at a position reachable by the biopsy forceps 500, a mark indicating the position of the region of interest is displayed in a display image. This makes it possible to notify the operator of a lesion, which is the target, at an appropriate timing during tissue sampling and to appropriately support the operator in a treatment.

### Modifications

### Modifications of Notification

The embodiment described above provides a configuration in which a mark indicating the position of a region of interest is displayed superimposed on an endoscopic image to give a notification that the region of interest is located at a position reachable by a treatment tool. However, the method for notification is not limited to that in this configuration. Modifications of the method for notification will be described hereinafter.

### (1) First Modification of Method for Notification

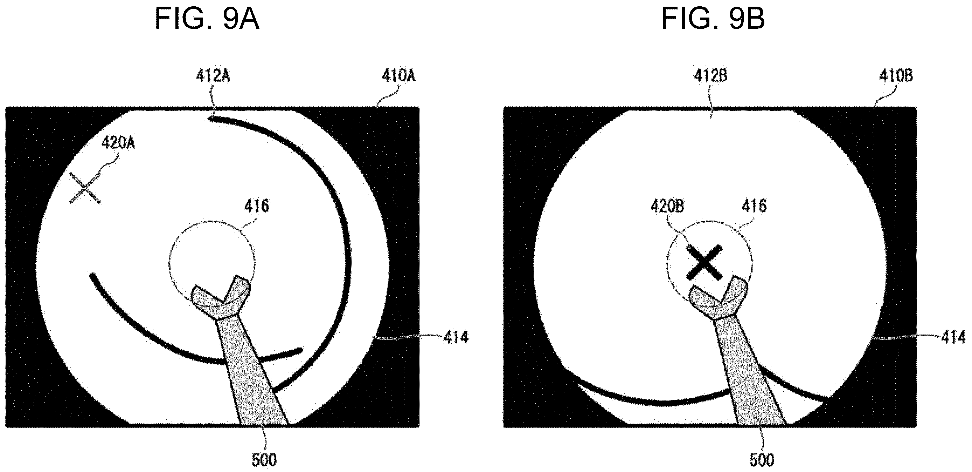

Figs. 9A and 9B are diagrams illustrating a first modification of display images. A display image 410A illustrated in Fig. 9A is a display image in a case where the region of interest is not located at a position reachable by the treatment tool 500. A display image 410B illustrated in Fig. 9B is a display image in a case where the region of interest is located at a position reachable by the treatment tool 500.

In this modification, as illustrated in Figs. 9A and 9B, upon detection of the region of interest from endoscopic images 412A and 412B, marks 420A and 420B indicating the position of the region of interest are displayed superimposed on the endoscopic images 412A and 412B.

However, the display forms of the marks 420A and 420B are different between the case where the region of interest is located at a position reachable by the treatment tool 500 and the case where the region of interest is not located at a position reachable by the treatment tool 500.

As illustrated in Fig. 9A, in the case where the region of interest is not located at a position reachable by the treatment tool 500, the mark 420A is displayed by a thin line. By contrast, in the case where the region of interest is located at a position reachable by the treatment tool 500, as illustrated in Fig. 9B, the mark 420B is displayed by a thick line. That is, the marks 420A and 420B are displayed with degrees of highlighting that are different between the case where the region of interest is located at a position reachable by the treatment tool 500 and the case where the region of interest is not located at a position reachable by the treatment tool 500. In the case where the region of interest is located at a position reachable by the treatment tool 500, the degree of highlighting of the mark 420B is high. Conversely, in the case where the region of interest is not located at a position reachable by the treatment tool 500, the degree of highlighting of the mark 420A is low.

As described above, the display form of the mark is changed between the case where the region of interest is located at a position reachable by the treatment tool and the case where the region of interest is not located at a position reachable by the treatment tool, thereby making it possible to provide a notification that the region of interest is located at a position reachable by the treatment tool.

This modification provides a configuration in which the display form of the mark is switched by changing the thickness of the lines of the mark. However, the method for switching the display form of the mark is not limited to that in this configuration. The display form of the mark can be switched by changing at least one of the color, shape, brightness, or line type of the mark. In this case, preferably, the display form of the mark is switched such that the degree of highlighting is increased in the case where the region of interest is located at a position reachable by the treatment tool.

Instead of this, a configuration can be used in which the mark is blinked to provide a notification in a case where the region of interest is located at a position reachable by the treatment tool.

The shape of the mark indicating the position of the region of interest is not limited to a cross (X), and various shapes can be employed. Alternatively, a configuration can be used in which a geometric figure (such as a rectangular frame) surrounding the region of interest is displayed as a geometric figure indicating the region of interest.

### (2) Second Modification of Method for Notification

The embodiment described above provides a configuration in which the display of the display region for the endoscopic image, which is set in the display image, is changed to provide a notification. In another configuration, the display of a portion other than the display region for the endoscopic image can be changed to provide a notification.

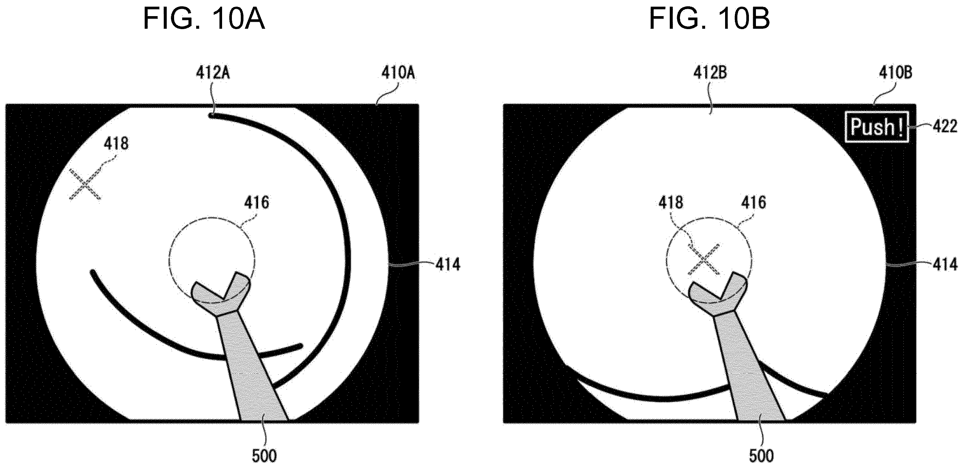

Figs. 10A and 10B are diagrams illustrating a second modification of display images. In Figs. 10A and 10B, a mark 418 of a cross (X) indicated by broken lines indicates the position of the region of interest. The mark 418 is displayed for convenience of description and is not displayed in an actual display image.

A display image 410A illustrated in Fig. 10A is a display image in a case where the region of interest is not located at a position reachable by the treatment tool 500. A display image 410B illustrated in Fig. 10B is a display image in a case where the region of interest is located at a position reachable by the treatment tool 500.

As illustrated in Fig. 10B, in a case where the region of interest is located at a position reachable by the treatment tool 500, a message 422 is displayed in a region outside the display region for the endoscopic image 412B. In this modification, the text "Push!" is displayed as the message 422. By contrast, in a case where the region of interest is not located at a position reachable by the treatment tool 500, as illustrated in Fig. 10A, the message is not displayed.

As described above, displaying the predetermined message 422 makes it possible to also provide a notification that the region of interest is located at a position reachable by the treatment tool.

This modification provides a configuration in which a message is displayed in a region other than the display region for the endoscopic image. However, the display position of the message is not limited to that in this configuration. Alternatively, a configuration can be used in which the message is displayed within the display region for the endoscopic image.

This modification further provides a configuration in which a message constituted by text is displayed to give a notification that the region of interest is located at a position reachable by the treatment tool. In another configuration, an icon or the like can be displayed to give a notification that the region of interest is located at a position reachable by the treatment tool. The message and the icon are examples of information.

### (3) Third Modification of Method for Notification

A configuration can be used in which audio is output to provide a notification in a case where the region of interest is located at a position reachable by the treatment tool. In this case, a speaker is separately included. The speaker outputs predetermined audio to give a notification that the region of interest is located at a position reachable by the treatment tool.

The notification using audio can be used in combination with a notification using display on the display 400.

### Modification of Determination of Whether Region of Interest Is Present at Position Reachable by Treatment Tool

The reference point P is set to a position at which the treatment is easily performed in consideration of the position of the protruded treatment tool, the type of the treatment tool, the content of the treatment, and so on. The threshold value r is also set in consideration of the type of the treatment tool, the content of the treatment, and so on.

The method for determining whether the region of interest is present at a position reachable by the treatment tool is not limited to the method described in the embodiment described above and is not a method falling within the scope of the claimed invention. For example, the distance to the region of interest may be measured, and whether the measured distance is less than or equal to a threshold value may be determined to determine whether the region of interest is present at a position reachable by the treatment tool. The distance to the region of interest is measured from the endoscopic image by using, for example, a known method for image measurement.

In the determination based on a reference point set in an endoscopic image, the threshold value r (first threshold value) can be set to be constant regardless of the type of the treatment tool, the type of the treatment, and the like, or can be set individually in accordance with the type of the treatment tool, the type of the treatment, and the like. For example, when the threshold value is set to be constant regardless of the type of the treatment tool, the type of the treatment, and the like, 1/10 of the width of the endoscopic image can be set as the threshold value. When the threshold value is to be changed in accordance with the type of the treatment tool, for example, the threshold value can be set in the following way. For example, 1/10 of the width of the endoscopic image is set as the threshold value for a treatment using forceps. For a treatment (local injection) using a local injection needle, 1/20 of the width of the endoscopic image is set as the threshold value. Since local injection is performed at a pinpoint, that is, at the root of a lesion, the threshold value is set so as to have a narrower range. For a treatment using a snare, 1/2 of the width of the endoscopic image is set as the threshold value. Since the snare can be widely opened to capture a lesion, the threshold value is set to have a wider range.

Alternatively, a configuration can be used in which the necessity of a notification is determined in consideration of the operating state of the endoscope and/or the treatment tool. For example, for a treatment using a snare, the necessity of a notification can be determined in consideration of information on the opening state of the snare. That is, whether the snare is opened with a size such that the region of interest can be sampled is determined to determine the necessity of a notification. In this case, the opening state of the snare can be detected from, for example, an image. Additionally, for example, for a treatment using a puncture needle with the protruding angle of the puncture needle adjustable, the necessity of a notification can be determined in consideration of information on the protruding angle.

### Second Embodiment

Here, an example in which the present disclosure is applied to an ultrasonic endoscope system will be described.

### System Configuration

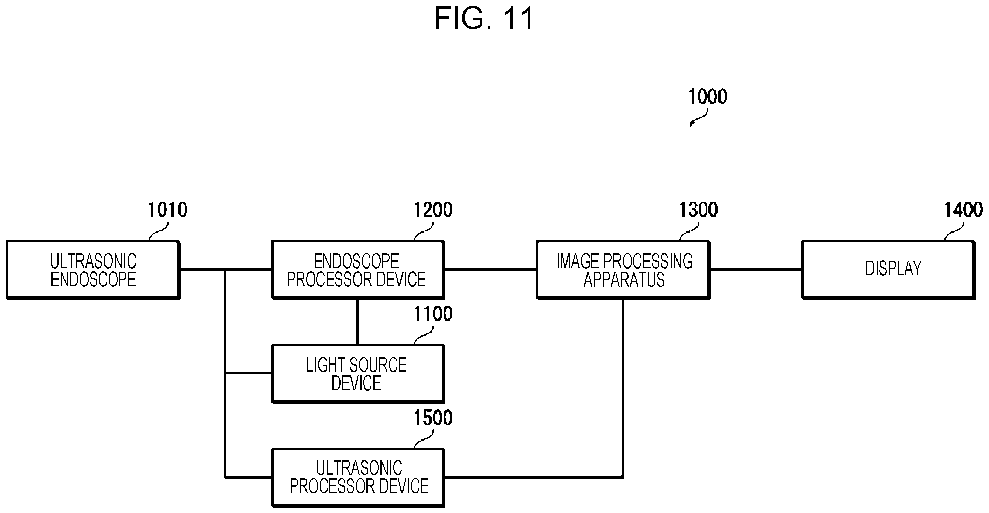

Fig. 11 is a block diagram illustrating an example of a system configuration of an ultrasonic endoscope system to which the present disclosure is applied.

As illustrated in Fig. 11, an ultrasonic endoscope system 1000 according to this embodiment includes an ultrasonic endoscope 1010, a light source device 1100, an endoscope processor device 1200, an ultrasonic processor device 1500, an image processing apparatus 1300, and a display 1400.

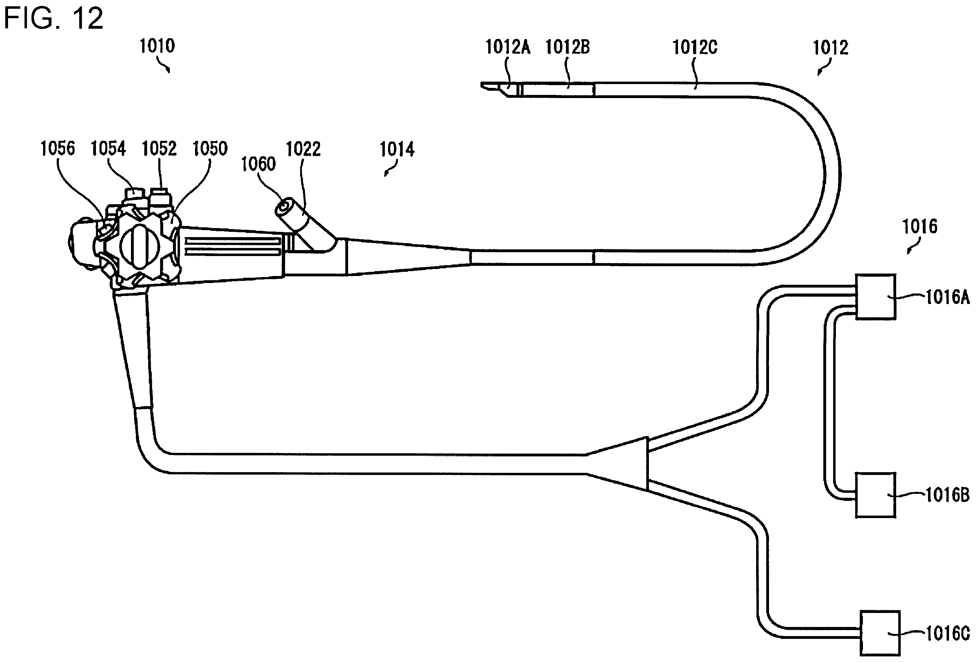

Fig. 12 is a diagram illustrating an example of the ultrasonic endoscope.

The ultrasonic endoscope 1010 illustrated in Fig. 12 is a convex ultrasonic endoscope and is mainly constituted by an insertion section 1012, an operation section 1014, and a connection section 1016.

The insertion section 1012 is constituted by, in order from the distal end side thereof, a tip part 1012A, a bending part 1012B that is bendable, and a soft part 1012C having flexibility.

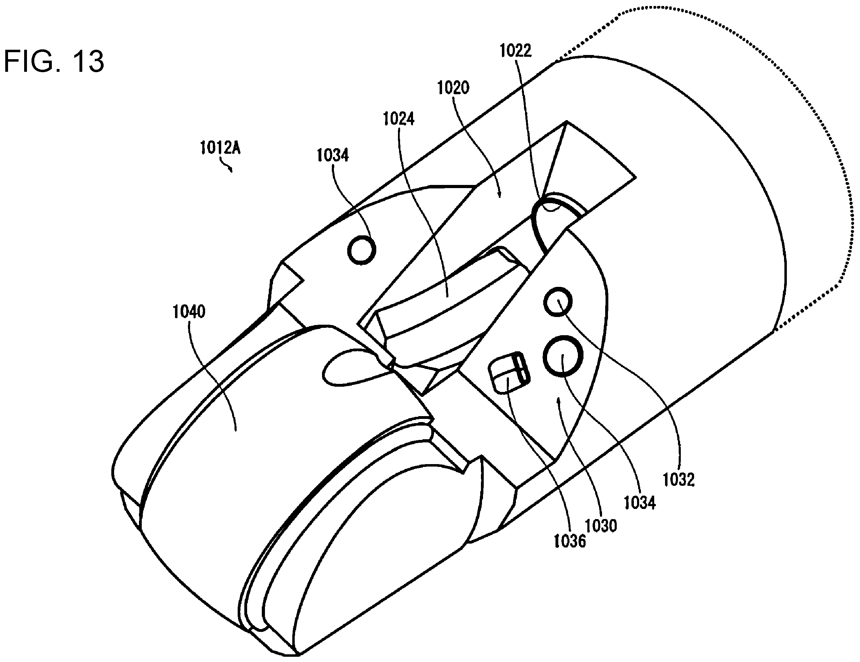

Fig. 13 is a perspective view illustrating an example configuration of a distal end of the insertion section of the ultrasonic endoscope.

As illustrated in Fig. 13, the tip part 1012A includes a treatment tool protruding portion 1020, an endoscopic observation portion 1030, and an ultrasound probe 1040.

The treatment tool protruding portion 1020 includes a treatment tool protruding port 1022 from which a treatment tool protrudes, an elevator 1024 that adjusts the protruding direction of the treatment tool, and so on. The elevator 1024 swings in accordance with the operation of an elevating lever 1056 included in the operation section 1014 to change the protruding angle of the treatment tool.

The endoscopic observation portion 1030 includes an observation window 1032, an illumination window 1034, a nozzle 1036, and so on. An imaging unit is included on the inside of the observation window 1032. The imaging unit is configured to include an imaging optical system and an image sensor.

The ultrasound probe 1040 has therein a plurality of piezoelectric elements that transmit and receive ultrasound waves, an acoustic lens, and so on.

As illustrated in Fig. 12, the operation section 1014 includes various operation members. The operation section 1014 includes, for example, an angle knob 1050 for bending operation of the bending part 1012B, a suction button 1052 for suction operation, an air/water supply button 1054 for air/water supply operation, the elevating lever 1056 to elevating operation of the elevator 1024, and so on.

As illustrated in Fig. 12, the operation section 1014 further includes a treatment tool insertion port 1060 through which the treatment tool is to be inserted. The treatment tool inserted from the treatment tool insertion port 1060 passes through a treatment tool channel (not illustrated) included inside the insertion section 1012 and protrudes from the treatment tool protruding port 1022 at the distal end.

The connection section 1016 is a portion for connecting the ultrasonic endoscope 1010 to the light source device 1100, the endoscope processor device 1200, and the ultrasonic processor device 1500. The connection section 1016 is constituted by a flexible cord. The connection section 1016 includes, at a distal end thereof, a connector 1016A for connecting to the light source device 1100, a connector 1016B for connecting to the endoscope processor device 1200, and a connector 1016C for connecting to the ultrasonic processor device 1500.

The light source device 1100 includes a light source and supplies light from the light source to the ultrasonic endoscope 1010 as illumination light. The illumination light supplied to the ultrasonic endoscope 1010 is emitted from the illumination window 1034 at the distal end through a light guide (not illustrated). The light from the light source is white light, for example.

The endoscope processor device 1200 captures an imaging signal output from the imaging unit of the ultrasonic endoscope 1010, performs predetermined signal processing, and generates an observation image (endoscopic image) obtained by the endoscopic observation portion 1030. Further, the endoscope processor device 1200 performs overall control of the entire system. The endoscope processor device 1200 is constituted by, for example, a computer including a CPU, a ROM, a RAM, and so on. In the endoscope processor device 1200, the CPU executes a predetermined program to implement a function of generating an endoscopic image, a function of performing overall control of the entire system, and so on. The ROM stores various programs to be executed by the CPU, data necessary for control, processing, and the like, and so on. The RAM provides a working memory space for the CPU.

The ultrasonic processor device 1500 captures an ultrasound imaging signal obtained via the ultrasound probe 1040 of the ultrasonic endoscope 1010, performs predetermined signal processing, and generates an ultrasound observation image (ultrasound image). The ultrasonic processor device 1500 is constituted by, for example, a computer including a CPU, a ROM, a RAM, and so on. In the ultrasonic processor device 1500, the CPU executes a predetermined program to implement a function of generating an ultrasound image, and so on. The ROM stores various programs to be executed by the CPU, data necessary for control, processing, and the like, and so on. The RAM provides a working memory space for the CPU.

The image processing apparatus 1300 performs a process of acquiring the endoscopic image output from the endoscope processor device 1200 and the ultrasound image output from the ultrasonic processor device 1500 and displaying the endoscopic image and the ultrasound image on the display 1400. Further, the image processing apparatus 1300 performs a process of supporting the operator in a predetermined treatment using the treatment tool through the display 1400. The image processing apparatus 1300 is constituted by, for example, a computer including a CPU, a ROM, a RAM, and so on. The image processing apparatus 1300 functions as an image processing apparatus in response to a predetermined program being executed by the CPU. The ROM stores various programs to be executed by the CPU, data necessary for various types of processing and control, and so on. The RAM provides a working memory space for the CPU.

With regard to the support of a treatment to be performed by the image processing apparatus 1300, the support of a treatment based on the endoscopic image is the same as that in the first embodiment described above. The support of a treatment based on an ultrasound image will be described here.

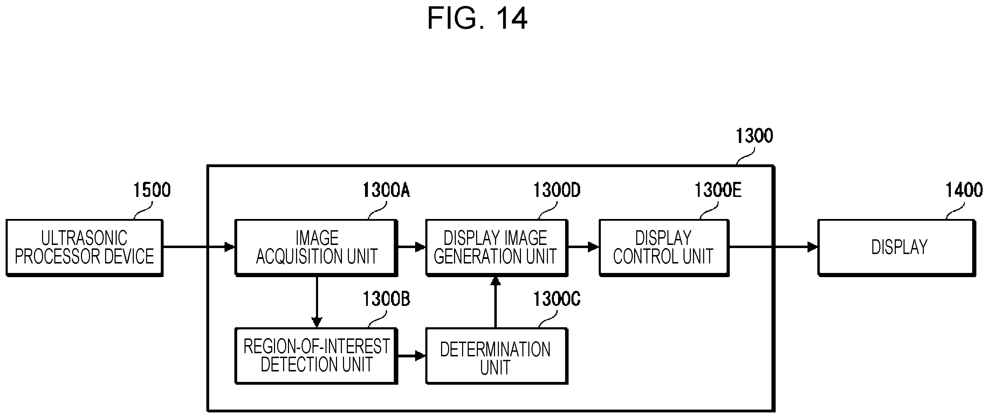

Fig. 14 is a block diagram of a function implemented by the image processing apparatus according to this embodiment.

As illustrated in Fig. 14, the image processing apparatus 1300 has the functions of an image acquisition unit 1300A, a region-of-interest detection unit 1300B, a determination unit 1300C, a display image generation unit 1300D, and a display control unit 1300E. These functions are implemented by the CPU executing a predetermined program (image processing program).

The image acquisition unit 1300A acquires an ultrasound image from the ultrasonic processor device 1500. The ultrasound image is a moving image. The image acquisition unit 1300A sequentially acquires images of respective frames constituting the moving image.

The region-of-interest detection unit 1300B detects a region of interest from the image of each of the frames acquired by the image acquisition unit 1300A. The term "region of interest", as used herein, refers to a region on which the treatment is to be performed. In the case of tissue sampling, the region of interest is a lesion portion, which is the target. The region-of-interest detection unit 1300B detects the region of interest from within the endoscopic image by, for example, image recognition. The image recognition can be performed using, for example, an image recognition model generated by machine learning (such as deep learning). Any other known method can be used.

The region of interest is detected by identification of the position of the region of interest in the image. The position is acquired as, for example, information on the position of the pixel at the center or centroid of the region of interest present in the endoscopic image.

Upon detection of a region of interest, the determination unit 1300C determines whether the region of interest is present at a position reachable by the treatment tool. The determination is performed in the following way.

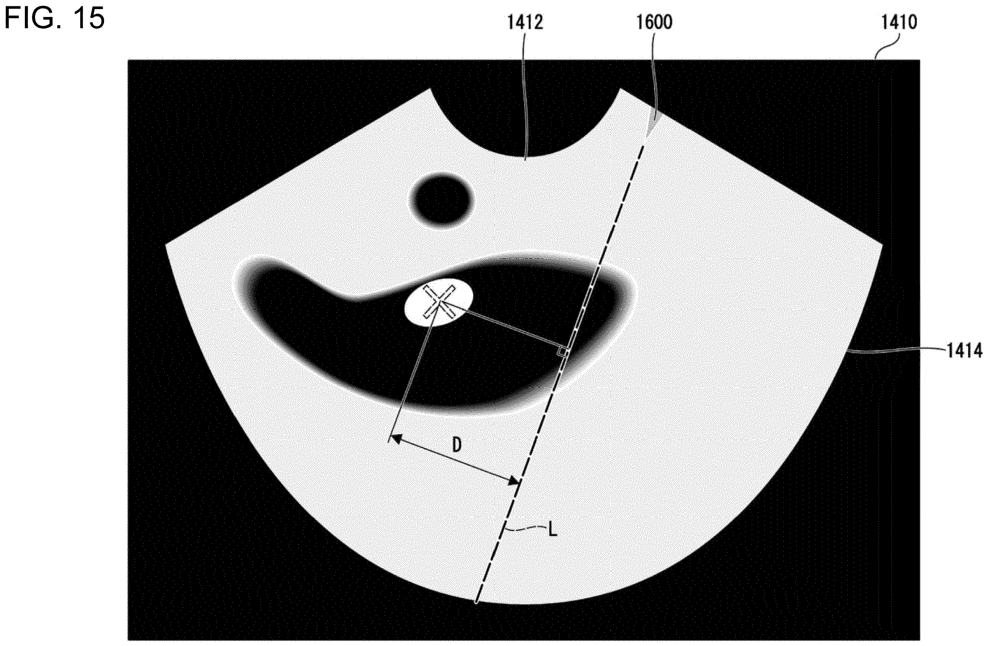

Fig. 15 is a diagram illustrating an example of a display image (an image to be displayed on the display) in a case where the treatment tool is protruded from the treatment tool protruding port. Fig. 15 illustrates an example of a case where tissue sampling is performed by using endoscopic ultrasound-fine needle aspiration (EUS-FNA).

As illustrated in Fig. 15, a display image 1410 on the display 1400 is constituted by a rectangular image having a predetermined aspect ratio. An ultrasound image 1412 is displayed in a predetermined display region 1414 set in the display image 1410. In the illustrated example, a sector-shaped region is set as the display region 1414 for the ultrasound image 1412.

When a treatment tool (puncture needle) 1600 is protruded from the treatment tool protruding port 1022, the treatment tool 1600 appears in the ultrasound image 1412. Since the positional relationship between the treatment tool protruding port 1022 and the ultrasound probe 1040 is fixed, the position at which the treatment tool 1600 appears is always constant. Its protruding direction is also constant. In Fig. 15, a straight line L indicated by a broken line is an extension line extending along the protruding direction of the treatment tool 1600 protruding from the treatment tool protruding port 1022. When the treatment tool 1600 is protruded from the treatment tool protruding port 1022, the treatment tool 1600 moves forward or backward along the straight line L in the ultrasound image 1412. The determination unit 1300C determines whether a distance D from the straight line L to the region of interest is less than or equal to a threshold value (less than or equal to a second threshold value) to determine whether the region of interest is present at a position reachable by the treatment tool 1600. The distance D from the straight line L to the region of interest is measured as the distance of a perpendicular line extending from the region of interest toward the straight line L. If the distance D is less than or equal to the threshold value, it is determined that the region of interest is present at a position reachable by the treatment tool 1600. On the other hand, if the distance D exceeds the threshold value, it is determined that the region of interest is not present at a position reachable by the treatment tool 1600.

The display image generation unit 1300D generates a display image to be displayed on the display 1400 from the ultrasound image acquired by the image acquisition unit 1300A. The display image generation unit 1300D generates the display image on the basis of a determination result of the determination unit 1300C. That is, if the determination unit 1300C determines that the region of interest is present at a position reachable by the treatment tool 1600, the display image is changed to notify the operator that the region of interest is present at a position reachable by the treatment tool 1600. Specifically, the display of the display region 1414 for the ultrasound image 1412 is changed.

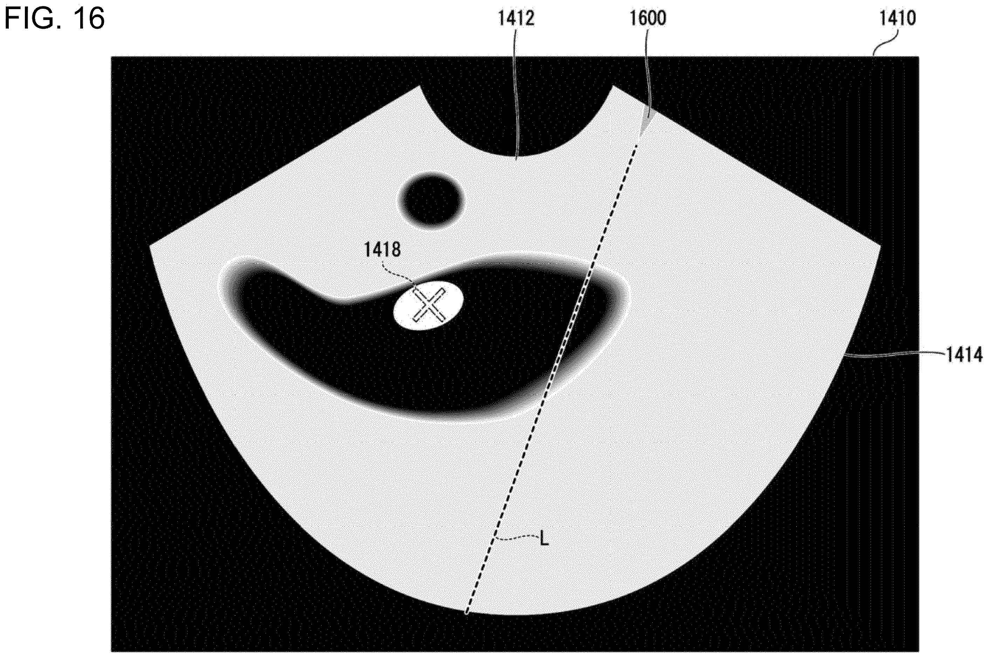

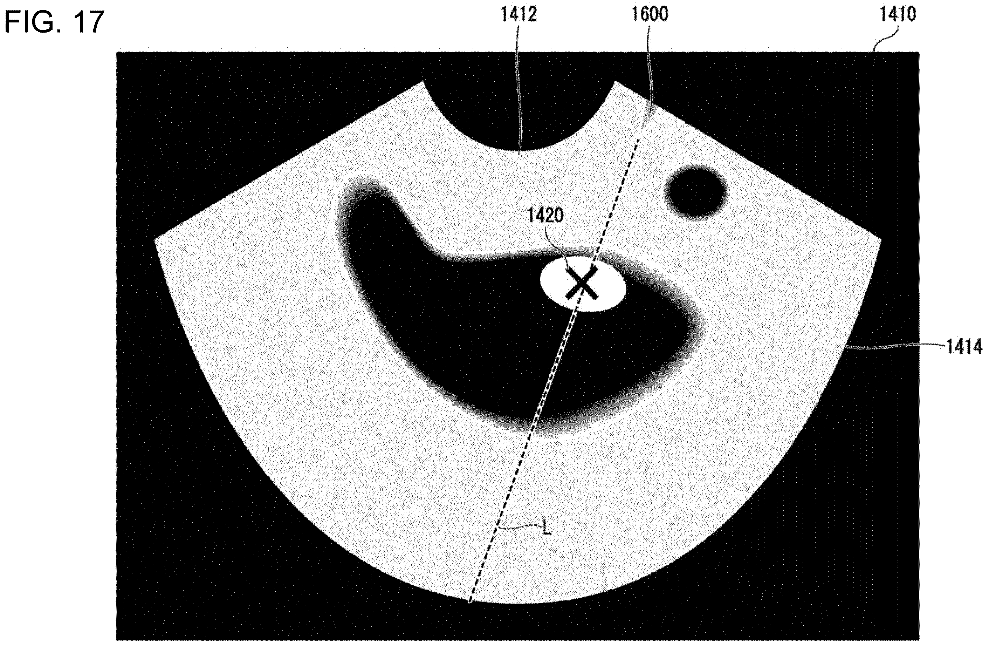

Fig. 16 and Fig. 17 are diagrams illustrating examples of a display image. Fig. 16 illustrates an example of a case where the region of interest is not present at a position reachable by the treatment tool 1600. Fig. 17 illustrates an example of a case where the region of interest is present at a position reachable by the treatment tool 1600.

In a case where the region of interest is not present at a position reachable by the treatment tool 1600, as illustrated in Fig. 16, only the ultrasound image 1412 is displayed in the display region 1414 for the ultrasound image 1412. In Fig. 16, a mark 1418 of a cross (X) indicated by broken lines indicates the position of the region of interest. In Fig. 16, the straight line L extending along the protruding direction of the treatment tool 1600 (the extension line of the treatment tool) and the mark 1418 indicating the position of the region of interest are displayed, for convenience of description; however, these are not actually displayed in the display image 1410.

By contrast, in a case where the region of interest is present at a position reachable by the treatment tool 1600, as illustrated in Fig. 17, in the display region 1414 for the ultrasound image 1412, a mark 1420 of a cross (X) indicating the position of the region of interest is displayed superimposed on the ultrasound image 1412. In Fig. 17, the straight line L extending along the protruding direction of the treatment tool 1600 (the extension line of the treatment tool) is displayed, for convenience of description; however, the straight line L is not actually displayed in the display image 1410.

The mark 1420 indicating the position of the region of interest is displayed only when the region of interest is present at a position reachable by the treatment tool 1600, thereby making it possible to appropriately notify the operator of the timing of the treatment.

The display control unit 1300E causes the display 400 to display the display image generated by the display image generation unit 1300D.

### Operation

Here, tissue sampling not according to the claimed invention using endoscopic ultrasound-fine needle aspiration will be described as an example. In tissue sampling using endoscopic ultrasound-fine needle aspiration, a puncture needle 1600 is used as the treatment tool. The puncture needle 1600 is inserted from the treatment tool insertion port 1060 included in the operation section 1014 of the ultrasonic endoscope 1010. The puncture needle 1600 inserted from the treatment tool insertion port 1060 protrudes from the treatment tool protruding port 1022 included at the distal end of the insertion section 1012. The puncture needle 1600, which is protruded, punctures the target to sample the tissue.

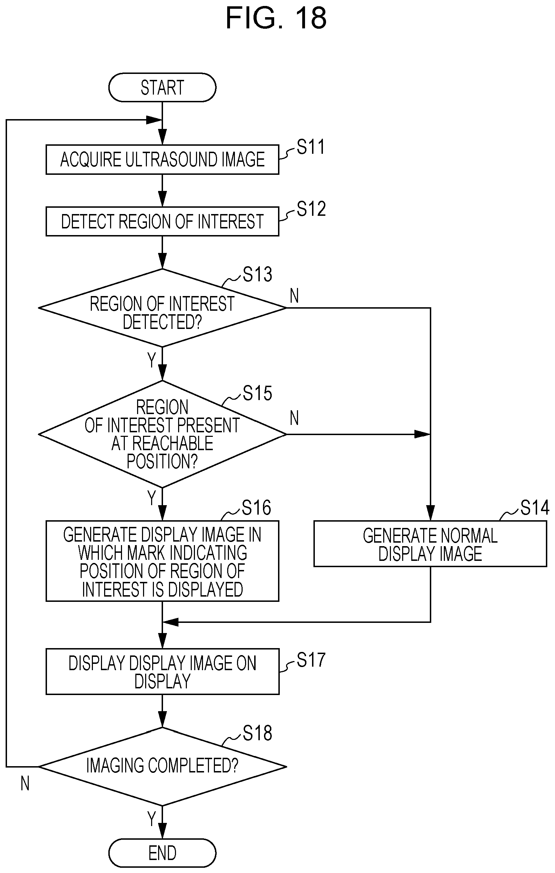

Fig. 18 is a flowchart illustrating a processing procedure for displaying an endoscopic image on the display by the image processing apparatus.

First, an ultrasound image is acquired from the ultrasonic processor device 1500 (step S11). Then, a region of interest is detected from the acquired ultrasound image (step S12). The region of interest used here is a lesion portion from which the tissue is to be sampled. Then, it is determined whether the region of interest has been detected on the basis of the detection result of the region of interest (step S13). If it is determined that the region of interest has not been detected, a normal display image is generated (step S14). Then, the generated display image is displayed on the display 1400 (step S17). The normal display image is an image in which a mark indicating the position of the region of interest is not displayed in the ultrasound image (an image with the display of the mark set to off) (see Fig. 16). On the other hand, if it is determined that the region of interest has been detected, it is further determined whether the region of interest is present at a position reachable by the puncture needle 1600 (step S15). If it is determined that the region of interest is not present at a position reachable by the puncture needle 1600, as in the case where the region of interest has not been detected, a normal display image is generated (step S14), and the generated display image is displayed on the display 1400 (step S17). On the other hand, if it is determined that the region of interest is present at a position reachable by the puncture needle 1600, a display image is generated in which the mark indicating the position of the region of interest is displayed (step S16). Then, the generated display image is displayed on the display 1400 (step S17). As illustrated in Fig. 17, the display image in which the mark indicating the position of the region of interest is displayed is an image in which the mark 1420 indicating the position of the region of interest is displayed superimposed on the ultrasound image 1412 (an image with the display of the mark set to on). Thereafter, it is determined whether ultrasound imaging with the ultrasonic endoscope 1010 is completed (step S18). In response to the completion of imaging, the display process ends. The completion of imaging is determined based on, for example, whether an image of the subsequent frame is input. If imaging is not completed, the process returns to step S11, and the series of processing operations described above is performed.

In the ultrasonic endoscope system 1000 according to this embodiment, as described above, when the region of interest is located at a position reachable by the puncture needle 1600, a mark indicating the position of the region of interest is displayed in a display image. This makes it possible to notify the operator of the target at an appropriate timing during tissue sampling and to appropriately support the operator.

### Modifications

### Modifications of Notification

The embodiment described above provides a configuration in which a mark indicating the position of a region of interest is displayed superimposed on an endoscopic image to give a notification that the region of interest is located at a position reachable by a treatment tool. However, the method for notification is not limited to that in this configuration. Modifications of the method for notification will be described hereinafter.

### (1) First Modification of Method for Notification

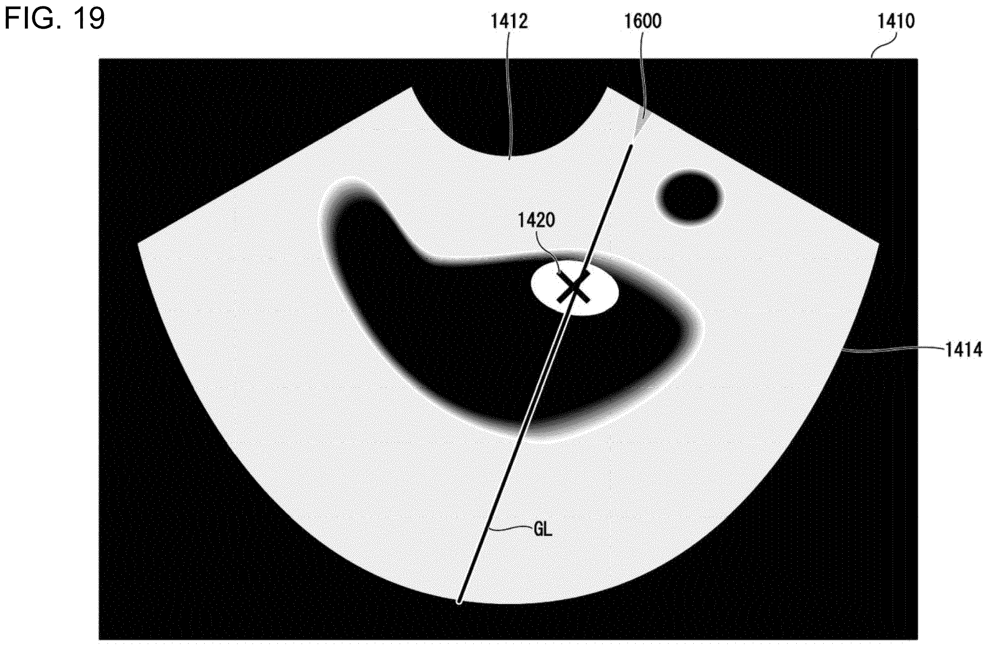

Fig. 19 is a diagram illustrating a first modification of a display image. Fig. 19 illustrates a display image in a case where the region of interest is located at a position reachable by the treatment tool.

As illustrated in Fig. 19, in this modification, when the region of interest is located at a position reachable by the treatment tool 1600, the mark 1420 indicating the position of the region of interest and a guide line GL indicating the protruding direction of the treatment tool 1600 are displayed superimposed on the ultrasound image 1412. The guide line GL is information indicating the protruding direction of the treatment tool 1600. When the treatment tool 1600 is protruded from the treatment tool protruding port 1022, the treatment tool 1600 moves along the guide line GL. The guide line GL matches the extension line of the treatment tool 1600 protruding from the treatment tool protruding port 1022.

As described above, the display of the guide line GL makes it possible to clarify the protruding direction of the treatment tool 1600 and to implement more satisfactory support.

This modification provides a configuration in which the mark 1420 and the guide line GL are displayed when the region of interest is located at a position reachable by a treatment tool. In another configuration, only the guide line GL can be displayed.

In still another configuration, the mark 1420 indicating the position of the region of interest can be constantly displayed, and the guide line GL can be displayed when the region of interest is located at a position reachable by a treatment tool. In this case, the display form of the mark 1420 may be changed between a case where the region of interest is located at a position reachable by the treatment tool and a case where the region of interest is not located at a position reachable by the treatment tool. For example, when the region of interest is located at a position reachable by the treatment tool, the color, shape, brightness, line type, or the like of the mark 1420 may be switched to change the degree of highlighting. That is, the degree of highlighting of the mark 1420 is increased in a case where the region of interest is located at a position reachable by the treatment tool.

In still another configuration, the guide line GL can be constantly displayed, and the mark 1420 can be displayed when the region of interest is located at a position reachable by a treatment tool. Also in this case, the display form of the guide line GL may be changed between a case where the region of interest is located at a position reachable by the treatment tool and a case where the region of interest is not located at a position reachable by the treatment tool. For example, when the region of interest is located at a position reachable by the treatment tool, the color, brightness, line type, or the like of the guide line GL may be switched to change the degree of highlighting. That is, the degree of highlighting of the guide line GL is increased in a case where the region of interest is located at a position reachable by the treatment tool.

Furthermore, the mark 1420 and the guide line GL may be constantly displayed, and the display forms of the mark 1420 and the guide line GL may be changed when the region of interest is located at a position reachable by a treatment tool.

Alternatively, a configuration can be used in which the guide line GL is constantly displayed regardless of whether the region of interest is detected.

The shape of the mark 1420 indicating the position of the region of interest is not limited to a cross (X), and various shapes may be employed.

### (2) Second Modification of Method for Notification

The embodiment described above provides a configuration in which the display of the display region for the ultrasound image is changed to provide a notification. Another configuration can be used in which the display of a portion other than the display region for the ultrasound image is changed to provide a notification.

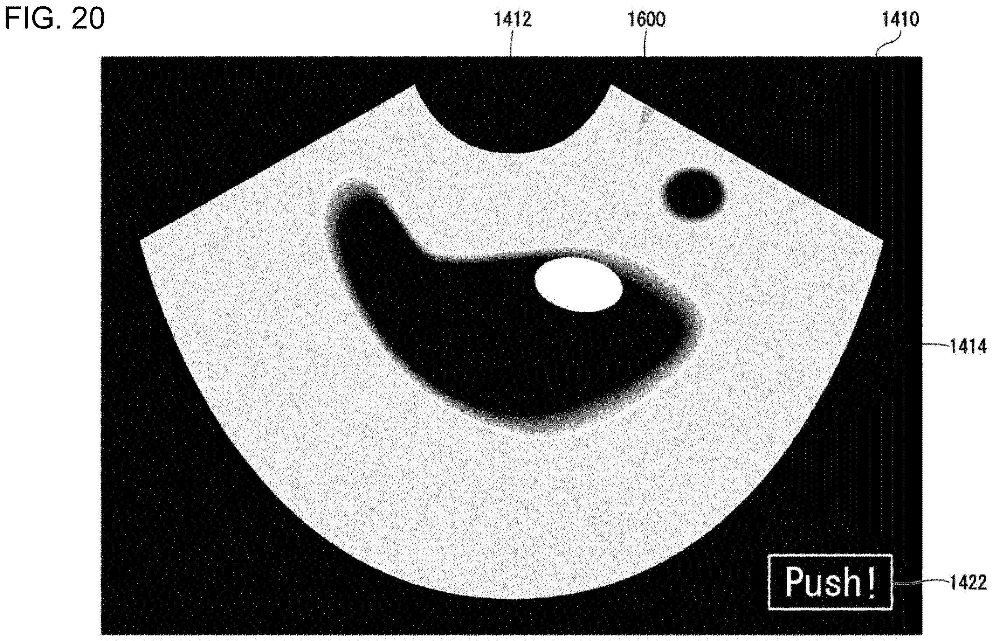

Fig. 20 is a diagram illustrating a second modification of a display image.

As illustrated in Fig. 20, in this modification, in a case where the region of interest is located at a position reachable by the treatment tool, a message 1422 is displayed in a region outside the display region 1414 for the ultrasound image 1412. In this modification, the text "Push!" is displayed as the message 1422.

As described above, displaying the predetermined message 1422 makes it possible to also provide a notification that the region of interest is located at a position reachable by the treatment tool.

This modification provides a configuration in which only the message 1422 is displayed when the region of interest is located at a position reachable by a treatment tool. In another configuration, a mark indicating the position of the region of interest and/or a guide line indicating the protrusion direction can be displayed at the same time as the display of the message 1422. In another configuration, the mark and/or the guide line can be constantly displayed. In still another configuration, in a case where the mark and/or the guide line is constantly displayed, the display form thereof can be switched between a case where the region of interest is located at a position reachable by the treatment tool and a case where the region of interest is not located at a position reachable by the treatment tool.

Further, this modification provides a configuration in which a message is displayed in a region other than a display region for an ultrasound image. However, the display position of the message is not limited to that in this configuration. Alternatively, a configuration can be used in which the message is displayed within the display region for the ultrasound image.

In still another configuration, an icon or the like can be displayed instead of the message.

### (3) Third Modification of Method for Notification

A configuration can be used in which audio is output to provide a notification in a case where the region of interest is located at a position reachable by the treatment tool. In this case, a speaker is separately included. The speaker outputs predetermined audio to give a notification that the region of interest is located at a position reachable by the treatment tool. The notification using audio can be used in combination with a notification using display on the display 400.

### Threshold Value (Second Threshold Value)

The threshold value (second threshold value) for determining whether the region of interest is located at a position reachable by a treatment tool may be set to be constant regardless of the type of the treatment tool, the type of the treatment, and the like, or may be set individually in accordance with the type of the treatment tool, the type of the treatment, and the like.

### Third Embodiment

The first and second embodiments described above provide a configuration in which, when the region of interest is located at a position reachable by a treatment tool, a mark or the like indicating the position of the region of interest is displayed to provide a notification to the operator. This embodiment provides a configuration in which, furthermore, it is determined whether an obstacle is present between the region of interest and the treatment tool and a notification is provided when no obstacle is present. In the following, a case where this processing is performed by an ultrasonic endoscope system will be described.

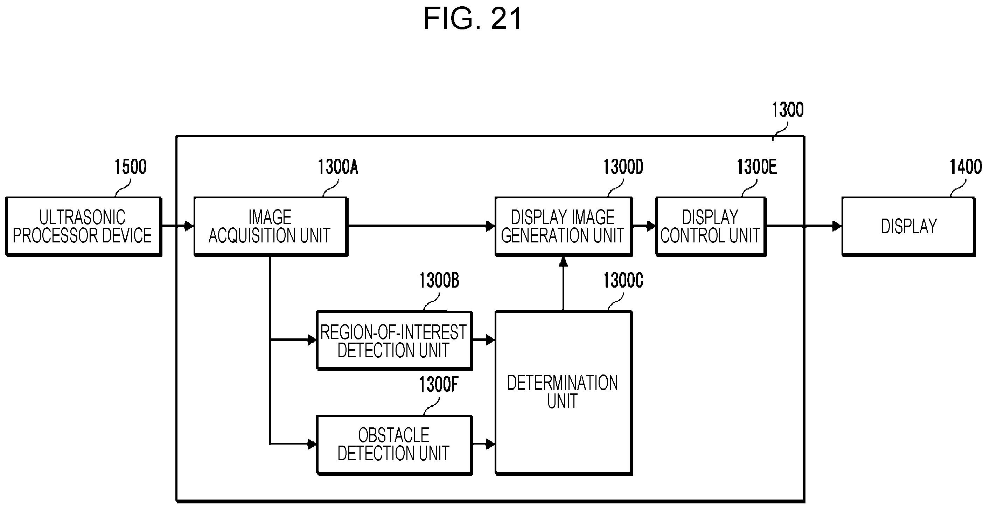

Fig. 21 is a block diagram of a function implemented by an image processing apparatus according to this embodiment.

As illustrated in Fig. 21, an image processing apparatus 1300 according to this embodiment further has a function of an obstacle detection unit 1300F in addition to the image processing apparatus according to the second embodiment described above.

The obstacle detection unit 1300F detects an obstacle from the ultrasound image acquired by the image acquisition unit 1300A. The term "obstacle", as used herein, refers to an object that obstructs the treatment tool 1600 protruded from the treatment tool protruding port 1022. Examples of the obstacle include large blood vessels. The obstacle detection unit 1300F detects the obstacle from within the ultrasound image by, for example, image recognition. The image recognition can be performed using, for example, an image recognition model generated by machine learning (such as deep learning). Any other known method can be used.

The obstacle is detected by identification of the position of the obstacle in the image. The position is acquired as, for example, information on the position of the pixel at the center or centroid of the obstacle present in the ultrasound image.

The determination unit 1300C determines the necessity of a notification on the basis of the detection results of the region-of-interest detection unit 1300B and the obstacle detection unit 1300F. Specifically, the determination unit 1300C determines to give a notification in a case where no obstacle is present between the region of interest and the distal end of the treatment tool and the region of interest is present at a position reachable by the treatment tool, and determines not to give a notification otherwise. Accordingly, even in a case where the region of interest is present at a position reachable by the treatment tool, it is determined not to give a notification if an obstacle is present between the distal end of the treatment tool and the region of interest.

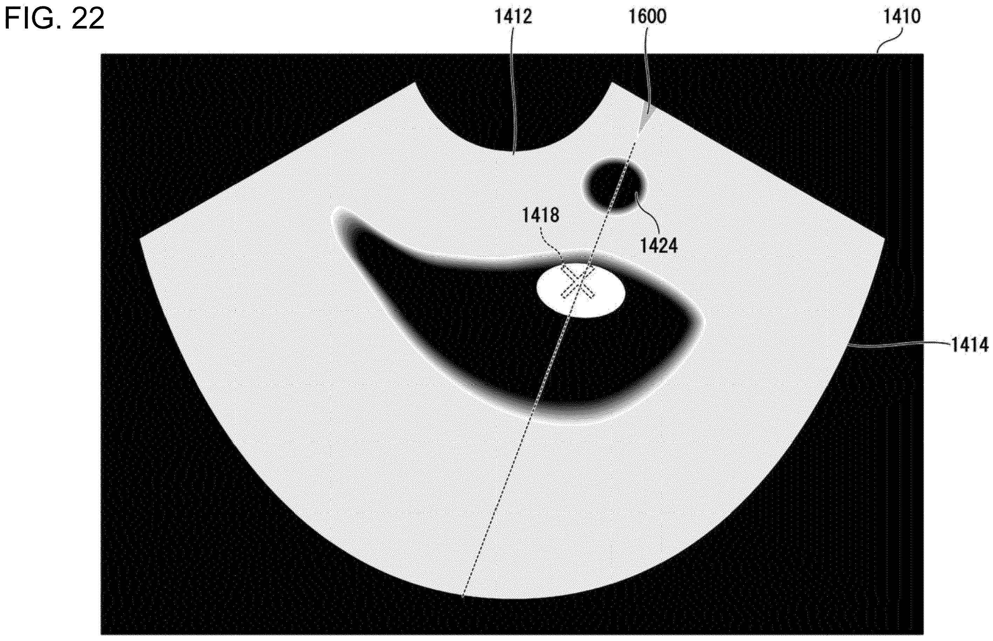

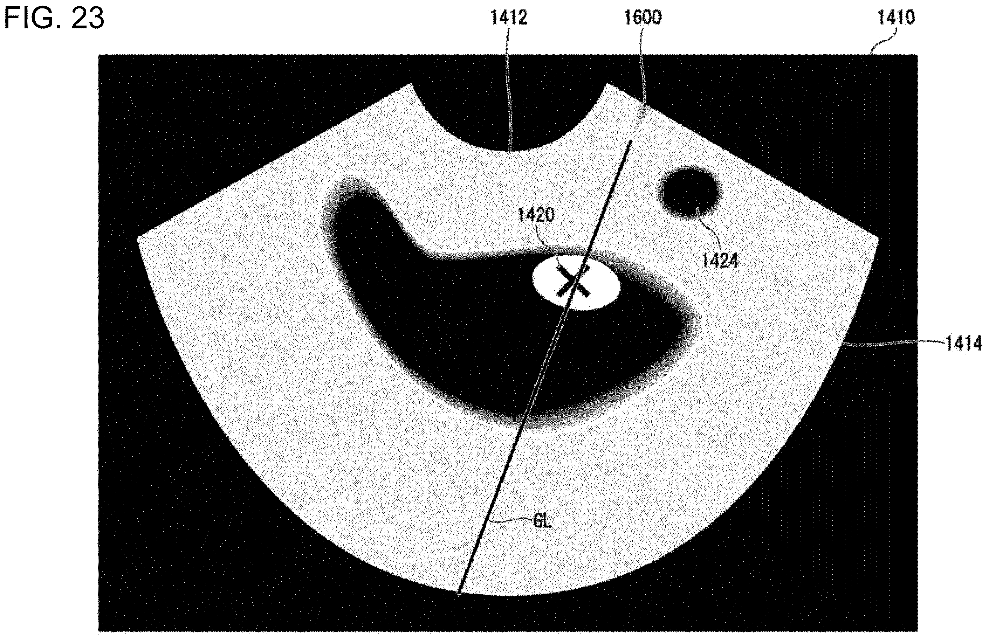

Fig. 22 and Fig. 23 are diagrams illustrating examples of a display image. In Fig. 22, a mark 1418 of a cross (X) indicated by broken lines indicates the position of the region of interest. In Fig. 22 and Fig. 23, furthermore, reference numeral 1424 denotes an obstacle (here, an artery). Fig. 22 illustrates an example of a case where an obstacle is present between the region of interest and the treatment tool 1600. Fig. 23 illustrates an example of a case where no obstacle is present between the region of interest and the treatment tool 1600 and the region of interest is present at a position reachable by the treatment tool 1600.

As illustrated in Fig. 22, in a case where the obstacle 1424 is present between the region of interest and the treatment tool 1600, only the ultrasound image 1412 is displayed in the display region 1414 for the ultrasound image 1412 even in a case where the region of interest is present at a position reachable by the treatment tool 1600. In Fig. 22, a straight line extending along the protruding direction of the treatment tool 1600 and a mark 1418 indicating the region of interest are displayed, for convenience of description; however, the straight line and the mark 1418 are not displayed in an actual display image.

By contrast, as illustrated in Fig. 23, when the obstacle 1424 is not present between the region of interest and the treatment tool 1600 and the region of interest is present at a position reachable by the treatment tool 1600, the guide line GL and the mark 1420 indicating the position of the region of interest are displayed superimposed on the ultrasound image 1412.

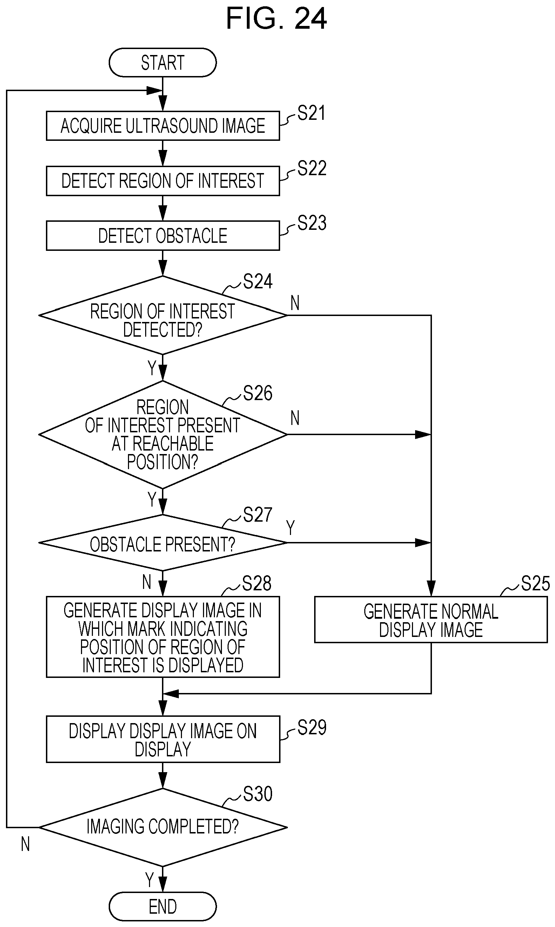

Fig. 24 is a flowchart illustrating a processing procedure for displaying an endoscopic image on the display by the image processing apparatus.

First, an ultrasound image is acquired from the ultrasonic processor device 1500 (step S21). Then, a region of interest is detected from the acquired ultrasound image (step S22). Then, an obstacle is detected from the acquired ultrasound image (step S23). The obstacle is, for example, an artery. Then, it is determined whether the region of interest has been detected on the basis of the detection result of the region of interest (step S24). If it is determined that the region of interest has not been detected, a normal display image is generated (step S25). Then, the generated display image is displayed on the display 1400 (step S29). On the other hand, if it is determined that the region of interest has been detected, it is further determined whether the region of interest is present at a position reachable by the puncture needle 1600 (step S26). If it is determined that the region of interest is not present at a position reachable by the puncture needle 1600, as in the case where the region of interest has not been detected, a normal display image is generated (step S25), and the generated display image is displayed on the display 1400 (step S29). On the other hand, if it is determined that the region of interest is present at a position reachable by the puncture needle 1600, the presence or absence of the obstacle is determined (step S27). That is, it is determined whether the obstacle is present between the distal end of the puncture needle 1600 and the region of interest. If it is determined that the obstacle is present, as in the case where the region of interest has not been detected and the case where it is determined that the region of interest is not present at a position reachable by the puncture needle 1600, a normal display image is generated (step S25), and the generated display image is displayed on the display 1400 (step S29). On the other hand, if it is determined that the obstacle is not present, a display image is generated in which the mark indicating the position of the region of interest is displayed (step S28). Then, the generated display image is displayed on the display 1400 (step S29). Thereafter, it is determined whether ultrasound imaging with the ultrasonic endoscope 1010 is completed (step S30). In response to the completion of imaging, the display process ends.

In the ultrasonic endoscope system 1000 according to this embodiment, as described above, a notification is provided only when a treatment is actually possible. This makes it possible to more appropriately support the operator.

While this embodiment provides a configuration in which a notification is given with the display of the guide line GL and the mark 1420, the form of the notification is not limited to this. For example, a configuration can be used in which either the guide line GL or the mark 1420 is displayed. In another configuration, a notification can be given with the display of the mark 1420 while the guide line GL is constantly displayed. In still another configuration, conversely, a notification can be given with the display of the guide line GL while the mark 1420 indicating the position of the region of interest is constantly displayed. Instead of this, a notification can be given in combination with audio notification, information display in a region other than the display region, and so on.

In this embodiment, processing in an ultrasonic endoscope has been described. Alternatively, a configuration can be used in which a typical endoscope determines the presence or absence of an obstacle in a similar manner and performs notification processing only when no obstacle is present.

### Fourth Embodiment

In this embodiment, it is determined whether the treatment tool is in a standby state from images (an endoscopic image and an ultrasound image), and notification processing is performed only when the treatment tool is in the standby state. The standby state refers to a state in which a treatment operation is available. In the following, a case where this processing is implemented in an ultrasonic endoscope system will be described as an example.

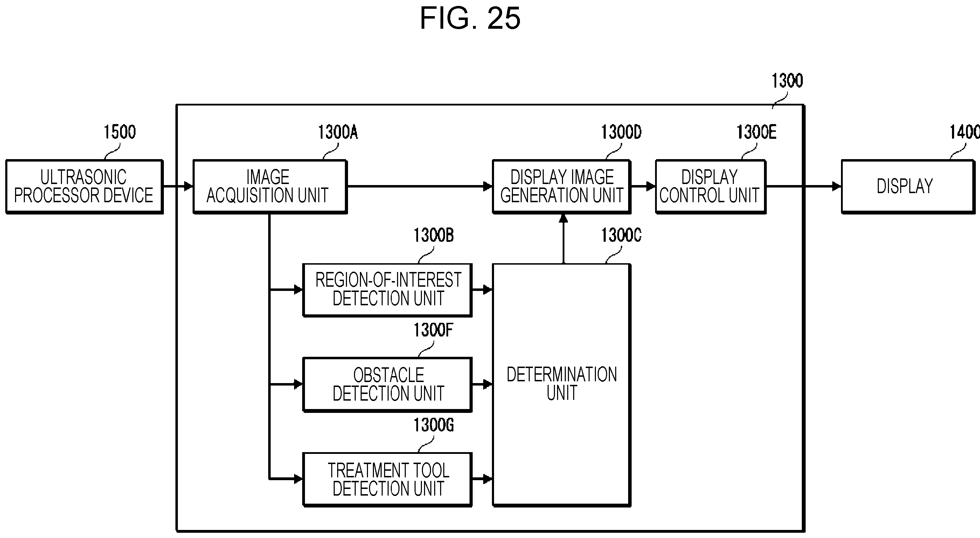

Fig. 25 is a block diagram of a function implemented by an image processing apparatus according to this embodiment.

As illustrated in Fig. 25, an image processing apparatus 1300 according to this embodiment further has a function of a treatment tool detection unit 1300G in addition to the image processing apparatus according to the third embodiment described above.

The treatment tool detection unit 1300G detects a treatment tool (for example, a puncture needle) from the ultrasound image acquired by the image acquisition unit 1300A. The treatment tool detection unit 1300G detects the treatment tool from within the ultrasound image by, for example, image recognition. The image recognition can be performed using, for example, an image recognition model generated by machine learning (such as deep learning). Any other known method can be used.

The determination unit 1300C determines the necessity of notification processing on the basis of the detection result of the treatment tool detection unit 1300G. Specifically, the determination unit 1300C determines that the notification processing is necessary in a case where the treatment tool 1600 has been detected.

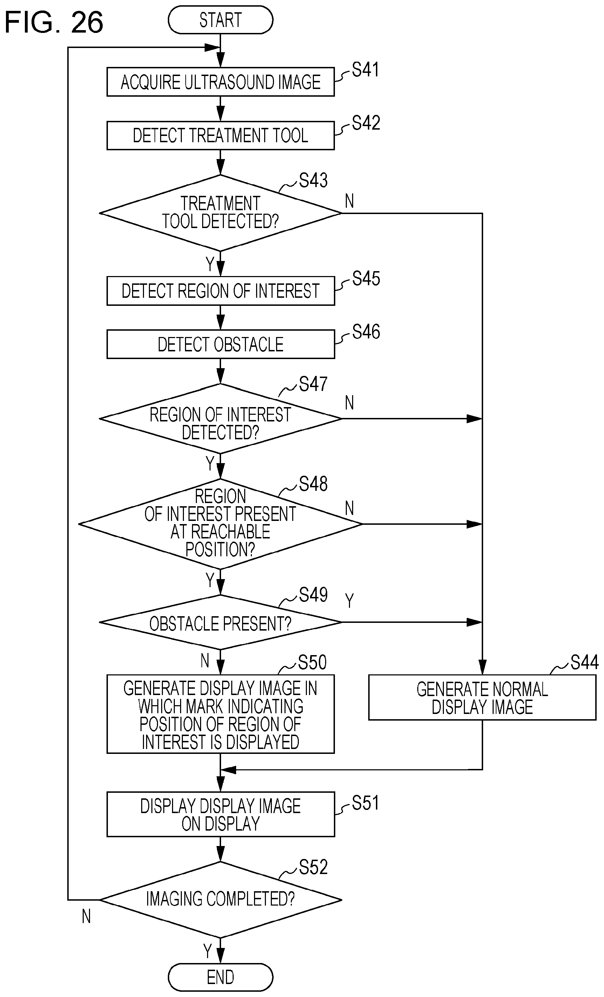

Fig. 26 is a flowchart illustrating a processing procedure for displaying an endoscopic image on the display by the image processing apparatus.

First, an ultrasound image is acquired from the ultrasonic processor device 1500 (step S41). Then, a treatment tool is detected from the acquired ultrasound image (step S42). Then, it is determined whether the treatment tool has been detected on the basis of the detection result of the treatment tool (step S42). That is, it is determined whether the treatment tool appears in the image. If it is determined that the treatment tool has not been detected, a normal display image is generated (step S44). Then, the generated display image is displayed on the display 1400 (step S51). On the other hand, if it is determined that the treatment tool has been detected, the region of interest is detected from the acquired ultrasound image (step S45). Then, an obstacle (for example, an artery) is detected from the acquired ultrasound image (step S46). Then, it is determined whether the region of interest has been detected on the basis of the detection result of the region of interest (step S47). If it is determined that the region of interest has not been detected, a normal display image is generated (step S44). Then, the generated display image is displayed on the display 1400 (step S51). On the other hand, if it is determined that the region of interest has been detected, it is further determined whether the region of interest is present at a position reachable by the puncture needle 1600 (step S48). If it is determined that the region of interest is not present at a position reachable by the puncture needle 1600, as in the case where the region of interest has not been detected, a normal display image is generated (step S44), and the generated display image is displayed on the display 1400 (step S51). On the other hand, if it is determined that the region of interest is present at a position reachable by the puncture needle 1600, the presence or absence of the obstacle is determined (step S49). If it is determined that the obstacle is present, as in the case where the region of interest has not been detected and the case where it is determined that the region of interest is not present at a position reachable by the puncture needle 1600, a normal display image is generated (step S44), and the generated display image is displayed on the display 1400 (step S51). On the other hand, if it is determined that the obstacle is not present, a display image is generated in which the mark indicating the position of the region of interest is displayed (step S50). Then, the generated display image is displayed on the display 1400 (step S51). Thereafter, it is determined whether ultrasound imaging with the ultrasonic endoscope 1010 is completed (step S52). In response to the completion of imaging, the display process ends.

In the ultrasonic endoscope system 1000 according to this embodiment, as described above, notification processing is performed only in the standby state. This makes it possible to more appropriately support the operator.

In this embodiment, an implementation in an ultrasonic endoscope system has been described as an example. Alternatively, a configuration can be used in which a typical endoscope system detects the standby state of a treatment tool in a similar manner and performs notification processing only in the standby state.

### Other Embodiments

### Applicable Treatment

In the embodiments described above, tissue sampling has been described as an example. Application of the present disclosure is not limited to this example. The present disclosure is applicable to a treatment performed with various instruments protruded from the distal end of an endoscope (including an ultrasonic endoscope). For example, in addition to tissue sampling in the embodiments described above, the present disclosure is also applicable to a treatment such as insertion of a guide wire in endoscopic ultrasound biliary drainage.

### Region of Interest

The region of interest is not limited to a lesion portion and may be a specific organ. For example, in the case of a typical endoscope, examples of the lesion portion serving as the region of interest include colonic polyp, gastric cancer, esophageal cancer, Barrett's esophagus, and ulcerative colitis. In the case of an ultrasonic endoscope, examples of the lesion portion include pancreatic cancer. In the case of an ultrasonic endoscope, examples of the organ serving as the region of interest include the common bile duct in endoscopic ultrasound biliary drainage.

### Hardware Configuration of Image Processing Apparatus

The functions of the image processing apparatus can be implemented by various processors. The various processors include a CPU that is a general-purpose processor configured to execute a program to function as various processing units, a Programmable Logic Device (PLD) that is a processor whose circuit configuration can be changed after manufacturing, such as an FPGA (Field Programmable Gate Array), a dedicated electric circuit that is a processor having a circuit configuration designed specifically for executing specific processing, such as an ASIC (Application Specific Integrated Circuit).

A single processing unit may be configured by one of the various processors or by two or more processors of the same type or different types. For example, the single processing unit may be configured by a plurality of FPGAs or a combination of a CPU and an FPGA. Alternatively, a plurality of processing units may be configured as a single processor. Examples of configuring a plurality of processing units as a single processor include, first, a form in which, as typified by a computer such as a client or a server, the single processor is configured as a combination of one or more CPUs and software and the processor functions as the plurality of processing units. The examples include, second, a form in which, as typified by a system on chip (SoC) or the like, a processor is used in which the functions of the entire system including the plurality of processing units are implemented as one IC (Integrated Circuit) chip. As described above, the various processing units are configured by using one or more of the various processors described above as a hardware structure.

More specifically, the hardware structure of the various processors is an electric circuit (circuitry) in which circuit elements such as semiconductor elements are combined.

The functions of the image processing apparatus can also be incorporated in a processor device constituting an endoscope system or an ultrasonic processor device constituting an ultrasonic endoscope system.

### Illumination Light

As the illumination light, light in various wavelength ranges according to the purpose of observation, such as white light, light in one or a plurality of specific wavelength ranges, or a combination thereof, is selected. The term "specific wavelength range" is a range narrower than the wavelength range of white. Specific examples of the specific wavelength range will be described below.

A first example of the specific wavelength range is, for example, the blue range or the green range in the visible range. The wavelength range in the first example includes a wavelength range of 390 nm or more and 450 nm or less or a wavelength range of 530 nm or more and 550 nm or less, and light in the first example has a peak wavelength in the wavelength range of 390 nm or more and 450 nm or less or in the wavelength range of 530 nm or more and 550 nm or less.

A second example of the specific wavelength range is, for example, the red range in the visible range. The wavelength range in the second example includes a wavelength range of 585 nm or more and 615 nm or less or a wavelength range of 610 nm or more and 730 nm or less, and light in the second example has a peak wavelength in the wavelength range of 585 nm or more and 615 nm or less, or in the wavelength range of 610 nm or more and 730 nm or less.

A third example of the specific wavelength range includes a wavelength range in which the light absorption coefficient is different between oxyhemoglobin and reduced hemoglobin, and light in the third example has a peak wavelength in the wavelength range in which the light absorption coefficient is different between oxyhemoglobin and reduced hemoglobin. The wavelength range in the third example includes a wavelength range of 400 ± 10 nm, a wavelength range of 440 ± 10 nm, a wavelength range of 470 ± 10 nm, or a wavelength range of 600 nm or more and 750 nm or less, and light in the third example has a peak wavelength in the wavelength range of 400 ± 10 nm, 440 ± 10 nm, 470 ± 10 nm, or 600 nm or more and 750 nm or less described above.

A fourth example of the specific wavelength range is the wavelength range of excitation light that is used for observation (fluorescence observation) of fluorescence emitted from a fluorescent substance in a living body and that excites the fluorescent substance, such as a wavelength range of 390 nm to 470 nm.

A fifth example of the specific wavelength range is the wavelength range of infrared light. The wavelength range in the fifth example includes a wavelength range of 790 nm or more and 820 nm or less or a wavelength range of 905 nm or more and 970 nm or less, and light in the fifth example has a peak wavelength in the wavelength range of 790 nm or more and 820 nm or less or in the wavelength range of 905 nm or more and 970 nm or less.

### Switching of Illumination Light

As the type of the light source, a laser light source, a xenon light source, an LED (Light -Emitting Diode) light source, or an appropriate combination thereof can be employed. The type and wavelength of the light source, the presence or absence of a filter, and so on are preferably configured in accordance with the type of the photographic subject, the purpose of observation, and so on. During observation, it is preferable to combine wavelengths of the illumination light and/or switch between wavelengths of the illumination light in accordance with the type of the photographic subject, the purpose of observation, and so on. In the switching of wavelengths, for example, a disk-shaped filter (rotary color filter) disposed in front of the light source and provided with a filter that transmits or blocks light having a specific wavelength may be rotated to switch the wavelength of light to be radiated.

### Imaging Unit

The image sensor included in the imaging unit of the endoscope is not limited to a color image sensor in which color filters are disposed for respective pixels, and may be a monochrome image sensor. In a case where a monochrome image sensor is used, imaging can be performed in a frame sequential (color sequential) manner by sequentially switching wavelengths of illumination light. For example, the wavelength of illumination light to be emitted may be sequentially switched among violet, blue, green, and red, or white light may be radiated and the wavelength of illumination light to be emitted may be switched by using a rotary color filter (red, green, blue, and the like). Alternatively, one or a plurality of narrow-band light rays may be radiated and the wavelength of illumination light to be emitted may be switched by using a rotary color filter. The narrow-band light rays may be infrared rays having two or more different wavelengths.

### Example Generation of Special-Light Image

The processor device may generate an image (so-called special-light image) having information on a specific wavelength range on the basis of an image (so-called normal-light image) obtained by imaging using white light. The processor device can acquire a signal in a specific wavelength range by performing an arithmetic operation based on color information of red (R), green (G), and blue (B) or cyan (C), magenta (M), and yellow (Y) included in the normal-light image.

### Program for Causing Computer to Implement Functions of Image Processing Apparatus

A program not according to the claimed invention for causing a computer to implement the functions of the image processing apparatus described in the embodiments described above can be recorded on a computer-readable medium, which is an optical disk, a magnetic disk, a semiconductor memory, or any other tangible non-transitory information storage medium, and the program can be provided via such an information storage medium. Instead of the program being stored in and provided by such a tangible non-transitory information storage medium, a program signal can be provided as a download service by using a telecommunications line such as the Internet.

Some or all of the functions of the image processing apparatus described in the embodiments described above can be provided as an application server to provide a service for providing processing functions via a telecommunications line.

### Combination of Embodiments, Modifications, and so on

The components described in the embodiments described above and the components described in the modifications can be used in appropriate combination, and some of the components can be replaced.

### Reference Signs List

1 endoscope system
10 endoscope
12 insertion section of endoscope
12A tip part of insertion section
12B bending part of insertion section
12C soft part of insertion section
14 operation section of endoscope
16 connection section of endoscope
16A connector of connection section
16B connector of connection section
20 observation window
22 illumination window
24 nozzle
26 forceps port
30 angle knob
32 air/water supply button
34 suction button
36 shutter release button
38 forceps insertion port
40 forceps channel
100 light source device
200 processor device
300 image processing apparatus
300A image acquisition unit
300B region-of-interest detection unit
300C determination unit
300D display image generation unit
300E display control unit
400 display
410 display image
410A display image
410B display image
412 endoscopic image
412A endoscopic image
412B endoscopic image
414 display region for endoscopic image
416 circle indicating range reachable by treatment tool
418 mark indicating position of region of interest
420 mark indicating position of region of interest
420A mark indicating position of region of interest
420B mark indicating position of region of interest
422 message
500 treatment tool (such as biopsy forceps)
510 insertion section of treatment tool
512 operation section of treatment tool
514 tip claw portion of treatment tool
512A handle of operation section
512B slider of operation section
1000 ultrasonic endoscope system
1010 ultrasonic endoscope
1012 insertion section of ultrasonic endoscope
1012Atip part of insertion section
1012B bending part of insertion section
1012C soft part of insertion section
1014 operation section of ultrasonic endoscope
1016 connection section of ultrasonic endoscope
1016Aconnector of connection section
1016B connector of connection section
1016C connector of connection section
1020 treatment tool protruding portion
1022 treatment tool protruding port
1024 elevator
1030 endoscopic observation portion
1032 observation window
1034 illumination window
1036 nozzle
1040 ultrasound probe
1050 angle knob
1052 suction button
1054 air/water supply button
1056 elevating lever
1060 treatment tool insertion port
1100 light source device
1200 endoscope processor device
1300 image processing apparatus
1300Aimage acquisition unit
1300B region-of-interest detection unit
1300C determination unit
1300D display image generation unit
1300E display control unit
1300F obstacle detection unit
1300Gtreatment tool detection unit
1400 display
1410 display image
1412 ultrasound image
1412B ultrasound image
1414 display region for ultrasound image
1418 mark indicating position of region of interest
1420 mark indicating position of region of interest
1422 message
1424 obstacle (such as artery)
1500 ultrasonic processor device
1600 treatment tool (such as puncture needle)
GL guide line
P reference point
S1 to S8 processing procedure for displaying endoscopic image on display by image processing apparatus
S11 to S18 processing procedure for displaying endoscopic image on display by image processing apparatus
S21 to S30 processing procedure for displaying endoscopic image on display by image processing apparatus
S41 to S52 processing procedure for displaying endoscopic image on display by image processing apparatus

## Claims

1. An image processing apparatus (300) for processing an image captured with an endoscope (10) having a distal end from which an instrument (500) is protrudable, the image processing apparatus (300) comprising a processor (200) configured to perform:
a process of acquiring an image captured with the endoscope (10);
a process of causing a display (400, 1400) to display the acquired image;
a process of detecting a region of interest from the acquired image, wherein the region of interest is a region on which a treatment is to be performed with the instrument (500);
a process of determining whether the region of interest is present at a position where the treatment is performable with the instrument (500) protruded from the distal end of the endoscope (10);
a process of determining whether an obstacle is present between the region of interest and the instrument (500) from the acquired image; and
a process of, in a case where it is determined that the region of interest is present at the position where the treatment is performable with the instrument (500) protruded from the distal end of the endoscope (10):
in a case where it is determined that the obstacle is not present between the region of interest and the instrument (500), providing a notification that the region of interest is present at the position where the treatment is performable with the instrument (500); and
in a case where it is determined that the obstacle is present between the region of interest and the instrument (500), not providing the notification.

2. The image processing apparatus (300) according to claim 1, wherein
the processor (200) is configured to determine whether a distance from a reference point set in the acquired image to the region of interest is less than or equal to a first threshold value to determine whether the region of interest is present at the position where the treatment is performable with the instrument (500) protruded from the distal end of the endoscope (10).

3. The image processing apparatus (300) according to claim 2, wherein
the first threshold value is set individually in accordance with at least one of a type of the instrument (500) or a type of the treatment performed with the instrument (500).

4. The image processing apparatus (300) according to claim 2 or 3, wherein
the reference point is a center of the image.

5. The image processing apparatus (300) according to claim 1, wherein
the processor (200) is configured to determine whether a distance from an extension line of the instrument (500) protruding from the distal end of the endoscope (10) to the region of interest is less than or equal to a second threshold value to determine whether the region of interest is present at the position where the treatment is performable with the instrument (500) protruded from the distal end of the endoscope (10).

6. The image processing apparatus (300) according to claim 5, wherein
the second threshold value is set individually in accordance with at least one of a type of the instrument (500) or a type of the treatment performed with the instrument (500).

7. The image processing apparatus (300) according to any one of claims 1 to 6, wherein
the processor (200) is configured to further perform a process of detecting the instrument (500) from the acquired image, and
the processor (200) is configured to perform the process of providing the notification in a case where the instrument (500) is detected from the acquired image, it is determined that the region of interest is present at the position where the treatment is performable with the instrument (500) protruded from the distal end of the endoscope (10) and it is determined that the obstacle is not present between the region of interest and the instrument (500).

8. The image processing apparatus (300) according to any one of claims 1 to 7, wherein
the processor (200) is configured to change a display image to be displayed on the display (400, 1400) to provide the notification.

9. The image processing apparatus (300) according to claim 8, wherein
the processor (200) is configured to change display of a display region for the image to provide the notification, the display region for the image being set in the display image.

10. The image processing apparatus (300) according to claim 9, wherein
the processor (200) is configured to display a geometric figure indicating the region of interest such that the geometric figure is superimposed on the image to be displayed in the display region to provide the notification.

11. The image processing apparatus (300) according to claim 8, wherein
the processor (200) is configured to further perform a process of displaying a geometric figure indicating the region of interest such that the geometric figure is superimposed on the image to be displayed in a display region for the image in a case where the region of interest is detected, the display region for the image being set in the display image, and
the processor (200) is configured to change display of the geometric figure to provide the notification, and preferably,
the processor (200) is configured to change at least one of a color, a shape, a brightness, or a line type of the geometric figure to change the display of the geometric figure.

12. The image processing apparatus (300) according to claim 8, wherein
the processor (200) is configured to further perform a process of causing information indicating a protruding direction of the instrument (500) to be displayed superimposed on the image to be displayed in a display region for the image, the display region for the image being set in the display image, and
the processor (200) is configured to change display of the information indicating the protruding direction of the instrument (500) to provide the notification.

13. The image processing apparatus (300) according to claim 12, wherein
the processor (200) is configured to display a straight line along the protruding direction of the instrument (500) as the information indicating the protruding direction of the instrument (500), and preferably,
the processor (200) is configured to change at least one of a color, a brightness, or a line type of the straight line to change display of the straight line.

14. The image processing apparatus (300) according to claim 8, wherein
the processor (200) is configured to change display of a region other than the display region for the image to provide the notification, the display region for the image being set in the display image.

## Patentansprüche

1. Bildverarbeitungsvorrichtung (300) zum Verarbeiten eines Bildes, das mit einem Endoskop (10) aufgenommen wird, das ein distales Ende, von dem ein Instrument (500) vorstehen kann, aufweist, wobei die Bildverarbeitungsvorrichtung (300) einen Prozessor (200) umfasst, der so konfiguriert ist, dass er durchführt:
einen Prozess des Erfassens eines mit dem Endoskop (10) aufgenommenen Bildes;
einen Prozess des Veranlassens einer Anzeige (400, 1400), das erfasste Bild anzuzeigen;
einen Prozess des Detektierens eines Bereichs von Interesse aus dem erfassten Bild, wobei der Bereich von Interesse ein Bereich ist, an dem eine Behandlung mit dem Instrument (500) durchgeführt werden soll;
einen Prozess des Bestimmens, ob der Bereich von Interesse an einer Position vorhanden ist, an der die Behandlung mit dem von dem distalen Ende des Endoskops (10) vorstehenden Instrument (500) durchgeführt werden kann;
einen Prozess des Bestimmens, ob ein Hindernis zwischen dem Bereich von Interesse und dem Instrument (500) aus dem erfassten Bild vorhanden ist; und
einen Prozess des, in einem Fall, in dem bestimmt wird, dass der Bereich von Interesse an der Position vorhanden ist, an der die Behandlung mit dem von dem distalen Ende des Endoskops (10) vorstehenden Instrument (500) durchgeführt werden kann:
in einem Fall, in dem bestimmt wird, dass das Hindernis zwischen dem Bereich von Interesse und dem Instrument (500) nicht vorhanden ist, Bereitstellens einer Benachrichtigung, dass der Bereich von Interesse an der Position vorhanden ist, an der die Behandlung mit dem Instrument (500) durchgeführt werden kann; und
in einem Fall, in dem bestimmt wird, dass das Hindernis zwischen dem Bereich von Interesse und dem Instrument (500) vorhanden ist, Nichtbereitstellens der Benachrichtigung.

2. Bildverarbeitungsvorrichtung (300) nach Anspruch 1, wobei
der Prozessor (200) so konfiguriert ist, dass er bestimmt, ob ein Abstand von einem in dem erfassten Bild eingestellten Referenzpunkt zu dem Bereich von Interesse kleiner oder gleich einem ersten Schwellenwert ist, um zu bestimmen, ob der Bereich von Interesse an der Position vorhanden ist, an der die Behandlung mit dem von dem distalen Ende des Endoskops (10) vorstehenden Instrument (500) durchgeführt werden kann.

3. Bildverarbeitungsvorrichtung (300) nach Anspruch 2, wobei
der erste Schwellenwert individuell in Übereinstimmung mit mindestens einem von einem Typ des Instruments (500) und einem Typ der mit dem Instrument (500) durchgeführten Behandlung eingestellt wird.

4. Bildverarbeitungsvorrichtung (300) nach Anspruch 2 oder 3, wobei
der Referenzpunkt ein Zentrum des Bildes ist.

5. Bildverarbeitungsvorrichtung (300) nach Anspruch 1, wobei
der Prozessor (200) so konfiguriert ist, dass er bestimmt, ob ein Abstand von einer Verlängerungslinie des Instruments (500), das von dem distalen Ende des Endoskops (10) vorsteht, zu dem Bereich von Interesse kleiner oder gleich einem zweiten Schwellenwert ist, um zu bestimmen, ob der Bereich von Interesse an der Position vorhanden ist, an der die Behandlung mit dem von dem distalen Ende des Endoskops (10) vorstehenden Instrument (500) durchgeführt werden kann.

6. Bildverarbeitungsvorrichtung (300) nach Anspruch 5, wobei
der zweite Schwellenwert individuell in Übereinstimmung mit mindestens einem von einem Typ des Instruments (500) und einem Typ der mit dem Instrument (500) durchgeführten Behandlung eingestellt wird.

7. Bildverarbeitungsvorrichtung (300) nach einem der Ansprüche 1 bis 6, wobei
der Prozessor (200) so konfiguriert ist, dass er ferner einen Prozess des Detektierens des Instruments (500) aus dem erfassten Bild durchführt, und
der Prozessor (200) so konfiguriert ist, dass er den Prozess des Bereitstellens der Benachrichtigung in einem Fall durchführt, in dem das Instrument (500) aus dem erfassten Bild detektiert wird, bestimmt wird, dass der Bereich von Interesse an der Position vorhanden ist, an der die Behandlung mit dem von dem distalen Ende des Endoskops (10) vorstehenden Instrument (500) durchgeführt werden kann, und bestimmt wird, dass das Hindernis nicht zwischen dem Bereich von Interesse und dem Instrument (500) vorhanden ist.

8. Bildverarbeitungsvorrichtung (300) nach einem der Ansprüche 1 bis 7, wobei
der Prozessor (200) so konfiguriert ist, dass er ein auf der Anzeige (400, 1400) anzuzeigendes Anzeigebild ändert, um die Benachrichtigung bereitzustellen.

9. Bildverarbeitungsvorrichtung (300) nach Anspruch 8, wobei
der Prozessor (200) so konfiguriert ist, dass er Anzeige eines Anzeigebereichs für das Bild ändert, um die Benachrichtigung bereitzustellen, wobei der Anzeigebereich für das Bild in dem Anzeigebild eingestellt ist.

10. Bildverarbeitungsvorrichtung (300) nach Anspruch 9, wobei
der Prozessor (200) so konfiguriert ist, dass er eine geometrische Figur, die den Bereich von Interesse angibt, so anzeigt, dass die geometrische Figur dem Bild, das in dem Anzeigebereich anzuzeigen ist, überlagert wird, um die Benachrichtigung bereitzustellen.

11. Bildverarbeitungsvorrichtung (300) nach Anspruch 8, wobei
der Prozessor (200) so konfiguriert ist, dass er ferner einen Prozess des Anzeigens einer geometrischen Figur, die den Bereich von Interesse angibt, so durchführt, dass die geometrische Figur dem Bild, das in einem Anzeigebereich für das Bild anzuzeigen ist, überlagert wird, in einem Fall, in dem der Bereich von Interesse detektiert wird, wobei der Anzeigebereich für das Bild in dem Anzeigebild eingestellt ist, und
der Prozessor (200) so konfiguriert ist, dass er Anzeige der geometrischen Figur ändert, um die Benachrichtigung bereitzustellen, und bevorzugt der Prozessor (200) so konfiguriert ist, dass er mindestens eines von einer Farbe, einer Form, einer Helligkeit und einem Linientyp der geometrischen Figur ändert, um die Anzeige der geometrischen Figur zu ändern.

12. Bildverarbeitungsvorrichtung (300) nach Anspruch 8, wobei
der Prozessor (200) so konfiguriert ist, dass er ferner einen Prozess des Veranlassens, dass Informationen, die eine Vorstehrichtung des Instruments (500) angeben, dem Bild, das in einem Anzeigebereich für das Bild anzuzeigen ist, überlagert angezeigt werden, durchführt, wobei der Anzeigebereich für das Bild in dem Anzeigebild eingestellt ist, und
der Prozessor (200) so konfiguriert ist, dass er Anzeige der Informationen, die die Vorstehrichtung des Instruments (500) angeben, ändert, um die Benachrichtigung bereitzustellen.

13. Bildverarbeitungsvorrichtung (300) nach Anspruch 12, wobei
der Prozessor (200) so konfiguriert ist, dass er eine gerade Linie entlang der Vorstehrichtung des Instruments (500) als die Informationen, die die Vorstehrichtung des Instruments (500) angeben, anzeigt, und bevorzugt
der Prozessor (200) so konfiguriert ist, dass er mindestens eines von einer Farbe, einer Helligkeit und einem Linientyp der geraden Linie ändert, um Anzeige der geraden Linie zu ändern.

14. Bildverarbeitungsvorrichtung (300) nach Anspruch 8, wobei
der Prozessor (200) so konfiguriert ist, dass er Anzeige eines anderen Bereichs, der von dem Anzeigebereich für das Bild verschieden ist, ändert, um die Benachrichtigung bereitzustellen, wobei der Anzeigebereich für das Bild in dem Anzeigebild eingestellt ist.

## Revendications

1. Appareil de traitement d'images (300) destiné à traiter une image capturée à l'aide d'un endoscope (10) comportant une extrémité distale à partir de laquelle un instrument (500) est apte à être déployé, l'appareil de traitement d'images (300) comprenant un processeur (200) configuré pour effectuer :
un processus consistant à acquérir une image capturée à l'aide de l'endoscope (10) ;
un processus consistant à commander à un affichage (400, 1400) d'afficher l'image acquise ;
un processus consistant à détecter une région d'intérêt à partir de l'image acquise, dans lequel la région d'intérêt est une région sur laquelle un traitement doit être effectué à l'aide de l'instrument (500) ;
un processus consistant à déterminer si la région d'intérêt est présente à une position où le traitement est apte à être effectué à l'aide de l'instrument (500) déployé à partir de l'extrémité distale de l'endoscope (10) ;
un processus consistant à déterminer si un obstacle est présent entre la région d'intérêt et l'instrument (500) à partir de l'image acquise ; et
un processus de, dans un cas où il est déterminé que la région d'intérêt est présente à la position où le traitement est apte à être effectué à l'aide de l'instrument (500) déployé à partir de l'extrémité distale de l'endoscope (10) :
dans un cas où il est déterminé que l'obstacle n'est pas présent entre la région d'intérêt et l'instrument (500), fourniture d'une notification indiquant que la région d'intérêt est présente à la position où le traitement est apte à être effectué à l'aide de l'instrument (500) ; et
dans un cas où il est déterminé que l'obstacle est présent entre la région d'intérêt et l'instrument (500), non-fourniture de la notification.

2. Appareil de traitement d'images (300) selon la revendication 1, dans lequel
le processeur (200) est configuré pour déterminer si une distance entre un point de référence défini dans l'image acquise et la région d'intérêt est inférieure ou égale à une première valeur seuil afin de déterminer si la région d'intérêt est présente à la position où le traitement est apte à être effectué à l'aide de l'instrument (500) déployé à partir de l'extrémité distale de l'endoscope (10).

3. Appareil de traitement d'images (300) selon la revendication 2, dans lequel
la première valeur seuil est définie individuellement en fonction d'au moins l'un parmi un type de l'instrument (500) ou un type de traitement effectué à l'aide de l'instrument (500).

4. Appareil de traitement d'images (300) selon la revendication 2 ou la revendication 3,
dans lequel
le point de référence est un centre de l'image.

5. Appareil de traitement d'images (300) selon la revendication 1, dans lequel
le processeur (200) est configuré pour déterminer si une distance entre une ligne de extension de l'instrument (500) déployé à partir de l'extrémité distale de l'endoscope (10) et la région d'intérêt est inférieure ou égale à une deuxième valeur seuil afin de déterminer si la région d'intérêt est présente à la position où le traitement est apte à être effectué à l'aide de l'instrument (500) déployé à partir de l'extrémité distale de l'endoscope (10).

6. Appareil de traitement d'images (300) selon la revendication 5, dans lequel
la deuxième valeur seuil est définie individuellement en fonction d'au moins l'un parmi un type de l'instrument (500) ou un type de traitement effectué à l'aide de l'instrument (500).

7. Appareil de traitement d'images (300) selon l'une quelconque des revendications 1 à 6, dans lequel
le processeur (200) est configuré pour effectuer en outre un processus consistant à détecter l'instrument (500) à partir de l'image acquise, et
le processeur (200) est configuré pour effectuer le processus consistant à fournir la notification dans un cas où l'instrument (500) est détecté à partir de l'image acquise, où il est déterminé que la région d'intérêt est présente à la position où le traitement est apte à être effectué à l'aide de l'instrument (500) déployé à partir de l'extrémité distale de l'endoscope (10), et où il est déterminé que l'obstacle n'est pas présent entre la région d'intérêt et l'instrument (500).

8. Appareil de traitement d'images (300) selon l'une quelconque des revendications 1 à 7,
dans lequel
le processeur (200) est configuré pour modifier une image d'affichage à afficher sur l'affichage (400, 1400) afin de fournir la notification.

9. Appareil de traitement d'images (300) selon la revendication 8, dans lequel
le processeur (200) est configuré pour modifier l'affichage d'une région d'affichage de l'image afin de fournir la notification, la région d'affichage de l'image étant définie dans l'image d'affichage.

10. Appareil de traitement d'images (300) selon la revendication 9, dans lequel
le processeur (200) est configuré pour afficher une figure géométrique indiquant la région d'intérêt de sorte que la figure géométrique soit superposée à l'image à afficher dans la région d'affichage afin de fournir la notification.

11. Appareil de traitement d'images (300) selon la revendication 8, dans lequel
le processeur (200) est configuré pour effectuer en outre un processus consistant à afficher une figure géométrique indiquant la région d'intérêt de sorte que la figure géométrique soit superposée à l'image à afficher dans une région d'affichage de l'image dans un cas où la région d'intérêt est détectée, la région d'affichage de l'image étant définie dans l'image d'affichage, et
le processeur (200) est configuré pour modifier l'affichage de la figure géométrique afin de fournir la notification, et de préférence,
le processeur (200) est configuré pour modifier au moins l'un parmi une couleur, une forme, une luminosité ou un type de ligne de la figure géométrique afin de modifier l'affichage de la figure géométrique.

12. Appareil de traitement d'images (300) selon la revendication 8, dans lequel
le processeur (200) est configuré pour effectuer en outre un processus consistant commander l'affichage des informations indiquant une direction de déploiement de l'instrument (500) superposées à l'image à afficher dans une région d'affichage de l'image, la région d'affichage de l'image étant définie dans l'image d'affichage, et
le processeur (200) est configuré pour modifier l'affichage des informations indiquant la direction de déploiement de l'instrument (500) afin de fournir la notification.

13. Appareil de traitement d'images (300) selon la revendication 12, dans lequel
le processeur (200) est configuré pour afficher une ligne droite le long de la direction de déploiement de l'instrument (500) en tant qu'informations indiquant la direction de déploiement de l'instrument (500), et de préférence,
le processeur (200) est configuré pour modifier au moins l'un parmi une couleur, une luminosité ou un type de ligne de la ligne droite afin de modifier l'affichage de la ligne droite.

14. Appareil de traitement d'images (300) selon la revendication 8, dans lequel
le processeur (200) est configuré pour modifier l'affichage d'une région autre que la région d'affichage de l'image afin de fournir la notification, la région d'affichage de l'image étant définie dans l'image d'affichage.
